# EUROPEAN PATENT SPECIFICATION

(11) **EP 2 520 653 B1**
(45) Date of publication and mention of the grant of the patent: **29.03.2017**
(21) Application number: 12166602.8
(22) Date of filing: 03.05.2012
(51) Int. Cl.: C12N 15/52, C12N 15/90

(54) **Integration of genes into the chromosome of saccharopolyspora spinosa**
Integration von Genen in das Chromosom von Saccharopolyspora spinosa
Intégration de gènes dans le chromosome de Saccharopolyspora spinosa

(30) Priority: 03.05.2011 US 201113100220; 03.05.2011 US 201113100202
(43) Date of publication of application: 07.11.2012
(73) Proprietor: Dow AgroSciences LLC, Indianapolis, IN 46268 (US)
(72) Inventor: Han, Lei, Carmel, IN 46032 (US); Mouncey, Nigel, Indianapolis, IN 46220 (US)
(74) Representative: Shah, Punita

(56) References cited:
- WO-A1-89/03883
- WO-A1-99/46387
- WO-A2-01/16303
- WO-A2-03/070908
- MATSUSHIMA P ET AL: "Conjugal transfer of cosmid DNA from Escherichia coli to Saccharopolyspora spinosa: effects of chromosomal insertions on macrolide A83543 production", GENE, ELSEVIER, AMSTERDAM, NL, vol. 146, no. 1, 19 August 1994 (1994-08-19), pages 39-45, XP023541380, ISSN: 0378-1119, DOI: 10.1016/0378-1119(94)90831-1 [retrieved on 1994-08-19]
- ZIRKLE ROSS ET AL: "Analysis of a 108-kb region of the Saccharopolyspora spinosa genome covering the obscurin polyketide synthase locus.", DNA SEQUENCE : THE JOURNAL OF DNA SEQUENCING AND MAPPING APR 2004 LNKD- PUBMED:15346767, vol. 15, no. 2, April 2004 (2004-04), pages 123-134, XP009161432, ISSN: 1042-5179
- WALDRON C ET AL: "A CLUSTER OF GENES FOR THE BIOSYNTHESIS OF SPINOSYNS, NOVEL MACROLIDE INSECT CONTROL AGENTS PRODUCED BY SACCHAROPOLYSPORA SPINOSA", ANTONIE VAN LEEUWENHOEK, DORDRECHT, NL, vol. 78, no. 3/04, 1 December 2000 (2000-12-01), pages 385-390, XP008015874, DOI: 10.1023/A:1010289901631
- KE-XUE HUANG ET AL: "Recent advances in the biochemistry of spinosyns", APPLIED MICROBIOLOGY AND BIOTECHNOLOGY, SPRINGER, BERLIN, DE, vol. 82, no. 1, 10 December 2008 (2008-12-10), pages 13-23, XP019705431, ISSN: 1432-0614
- KIRST H A ET AL: "DISCOVERY, ISOLATION, AND STRUCTURE ELUCIDATION OF A FAMILY OF STRUCTURALLY UNIQUE, FERMENTATION-DERIVED TETRACYCLIC MACROLIDES", WATER-SOLUBLE POLYMERS: SYNTHESIS, SOLUTION PROPERTIES AND APPLICATIONS, AMERICAN CHEMICAL SOCIETY, WASHINGTON, DC, US, 1 January 1992 (1992-01-01), pages 214-225, XP000944494, ISBN: 978-0-541-23408-9
- SHEEHAN L S ET AL: "Engineering of the Spinosyn PKS: Directing Starter Unit Incorporation", JOURNAL OF NATURAL PRODUCTS, AMERICAN CHEMICAL SOCIETY, US, vol. 69, no. 12, 27 December 2006 (2006-12-27), pages 1702-1710, XP002566660, ISSN: 0163-3864, DOI: 10.1021/NP0602517 [retrieved on 2006-12-08]

## Description

### TECHNICAL FIELD

The invention applies to the technical field of molecular genetics wherein genes may be integrated into the chromosome of *Saccharopolyspora spinosa.* A key metabolic engineering approach is the integration and expression of target genes at chromosomal DNA regions that result in little to no negative impact on spinosyn production or growth.

### BACKGROUND

As disclosed in U.S. Pat. No. 5,362,634, fermentation product A83543 is a family of related compounds produced by *Saccharopolyspora spinosa.* The known members of this family have been referred to as factors or components, and each has been given an identifying letter designation. These compounds are hereinafter referred to as spinosyn A, B, etc. The spinosyn compounds are useful for the control of arachnids, nematodes and insects, in particular, Lepidoptera and Diptera species. The compounds are considered environmentally friendly with an appealing toxicological profile.

The naturally produced spinosyn compounds are macrolides consisting of a 21-carbon tetracyclic lactone to which are attached two deoxysugars, a neutral sugar (rhamnose) and an amino sugar (forosamine) (see Kirst et al., (1991). If the amino sugar is not present, the compounds have been referred to as the pseudoaglycone of A, D, etc., and if the neutral sugar is not present then the compounds have been referred to as the reverse pseudoaglycone of A, D, etc. A more preferred nomenclature is to refer to the pseudoaglycones as spinosyn A 17-Psa, spinosyn D 17-Psa, etc., and to the reverse pseudoaglycones as spinosyn A 9-Psa, spinosyn D 9-Psa, etc.

The naturally produced spinosyn compounds may be produced via fermentation from *S. spinosa strains* NRRL 18395, 18537, 18538, 18539, 18719, 18720, 18743 and 18823 and derivatives therefrom. These cultures have been deposited and made part of the stock culture collection of the Midwest Area Northern Regional Research Center, Agricultural Research Service, United States Department of Agriculture, 1815 North University Street, Peoria, III., 61604.

U.S. Pat. No. 5,362,634 and corresponding European Patent No. 0375316 B1 relate to spinosyns A, B, C, D, E, F, G, H, and J. These compounds are said to be produced by culturing a strain of the novel microorganism *Saccharopolyspora spinosa* selected from NRRL 18395, NRRL 18537, NRRL 18538, and NRRL 18539.

WO 93/09126 relates to spinosyns L, M, N, Q, R, S, and T. Also discussed therein are two spinosyn J producing strains: NRRL 18719 and NRRL 18720, and a strain that produces spinosyns Q, R, S, and T: NRRL 18823.

WO 94/20518 and U.S. Pat. No. 5,670,486 relates to spinosyns K, O, P, U, V, W, and Y, and derivatives thereof. Also discussed therein is spinosyn K-producing strain NRRL 18743.

Matsushima P et al., 1994 relates to the conjugal transfer of cosmid DNA from *E.coli* to *S.spinosa* and the effects of chromosomal insertions on macrolide A83543 production.

WO 99/46387 relates to the cloning of the spinosyn gene cluster and various modifications thereof.

Zirkle Ross et al., 2004 relates to the analysis of a 108-kb region of the *S.spinosa* genome covering the obscurin polyketide synthase locus.

It would be desirable to improve heterologous gene expression in *S. spinosa.*

### DISCLOSURE OF THE INVENTION

The present invention provides a method for identifying and validating a neutral site in the *S*. *spinosa* genome, wherein a polynucleotide(s) can be integrated at said site and stably expressed.

In an embodiment, the polynucleotide(s) may contain at least one gene expression cassette, which can be integrated at said neutral site and stably expressed.

In an embodiment, the polynucleotide can be expressed over subsequent generations.

In an embodiment, the obscurin polyketide synthase (PKS) locus may be used as a neutral site for integration of a polynucleotide, either foreign or native, within the genome of *S*. *spinosa.* More specifically the *obsA* gene of the obscurin polyketide synthase (PKS) locus can be used as a neutral site, as this gene can be disrupted without negatively impacting spinosyn production, growth or other desired metabolic characteristics.

Thus, the present invention provides a method of stably expressing a polynucleotide in *S*. *spinosa,* comprising:
a) identifying and validating a neutral site in the *S. spinosa* genome, wherein a neutral site is one which can be disrupted without negatively impacting spinosyn production, *S. spinosa* growth or a desired metabolic characteristic;
b) introducing at said neutral site, a polynucleotide.

In an embodiment, the polynucleotide is heterologous. In an embodiment, the polynucleotide comprises at least one gene expression cassette. In an embodiment, the polynucleotide may be expressed stably over subsequent generations.

Additional methods of the present invention include polynucleotide integration into chromosomal DNA of *S. spinosa* species, which are useful for the production of insecticides, integrants thereof, and also to the use of the integrants.

Thus, the present invention also provides a genetically modified *S. spinosa* host cell comprising a polynucleotide integrated at a neutral site in the *S. spinosa* genome. Preferably, the host cell is generated by a method of the invention. A host cell of the invention may be a descendent of a host cell generated by a method of the invention, and which is capable of stably expressing the polynucleotide. In an embodiment, the neutral site is the obscurin polyketide synthase (PKS) locus. In an embodiment, the neutral site is the *obsA* gene. Thus, a genetically modified host cell of the invention may comprise a disrupted obscurin polyketide synthase (PKS) locus, preferably a disrupted *obsA* gene. In an embodiment, the polynucleotide is heterologous. In an embodiment, the polynucleotide comprises at least one gene expression cassette. In an embodiment, the polynucleotide may be expressed stably over subsequent generations.

The present invention also provides the use of a genetically modified *S*. *spinosa* host cell comprising a polynucleotide integrated at a neutral site in the *S. spinosa* genome, for stable expression. In an embodiment, the host cell is as defined herein. In an embodiment, the host cell is used for the recombinant expression of a gene product. In an embodiment the host cell is used for the improved expression of a spinosyn, preferably A/D and/or J/L.

Other embodiments of the present invention can include integrating a polynucleotide into the *S. spinosa* genome, the expression of which results in increased spinosyn production. The methods, host cells and uses of the invention may provide for the expression of a spinosyn enhancing gene. Thus, in embodiments, the polynucleotide integrated into a neutral site may comprise a spinosyn enhancing gene. In an embodiment, the spinosyn enhancing gene may encode an oxygen binding protein. In an embodiment, the oxygen binding protein is a hemoglobin. In an embodiment, the oxygen binding protein is *Vitreoscilla* hemoglobin (VHb), and the spinosyn enhancing gene is the gene encoding *Vitreoscilla* hemoglobin.

Other embodiments of the present invention can include integrating a polynucleotide into the *S. spinosa* genome without negatively impacting spinosyn production, growth or other desired metabolic characteristics

Other embodiments of the invention provide for the expression of genes encoding spinosyn biosynthetic enzymes. Such embodiments enable the production of novel spinosyns, derivatives of native spinosyns, or altered (preferably improved) expression of native spinosyns. This may improve the spectrum of insecticidal activity and/or increase the titer levels of spinosyns, and/or impart new beneficial characteristics to the existing spinosyn production strains. Thus, embodiments of the present invention can include a method of improving spinosyn production, comprising integrating into a neutral site of the *S. spinosa* genome, a polynucleotide, wherein the polynucleotide comprises a gene encoding a spinosyn biosynthetic enzyme. Also provided is a genetically modified host cell, comprising a polynucleotide comprising a gene encoding a spinosyn biosynthetic enzyme integrated into a neutral site of the host cell genome. Also provided is the use of a host cell as defined herein in the improved production of a spinosyn. In an embodiment, the neutral site is as defined herein.

In embodiments, the invention provides for the integration of two or more spinosyn enhancing genes and/or genes encoding spinosyn biosynthetic enzymes. In an embodiment, at least one is provided at a neutral site in the host cell genome. Any additional heterologous polynucleotides may be provided either at the same neutral site, at a different genomic location, or as a non-integrated expression construct.

The present invention also includes within its scope a method of producing a novel spinosyn or a spinosyn intermediate or derivative, in an *S. spinosa* host cell, comprising integrating a polynucleotide into chromosomal DNA of a *S*. *spinosa* host cell, wherein the polynucleotide is capable of disrupting expression of a native gene encoding a spinosyn biosynthetic enzyme. Thus, in an embodiment, the method of stably expressing a polynucleotide in *S. spinosa* comprises c) integrating a polynucleotide into chromosomal DNA of a *S*. *spinosa* host cell, wherein the polynucleotide is capable of disrupting expression of a native gene encoding a spinosyn biosynthetic enzyme. The present invention also provides a genetically modified *S. spinosa* host cell comprising a polynucleotide integrated therein, wherein the polynucleotide is capable of disrupting expression of a native gene encoding a spinosyn biosynthetic enzyme. In an embodiment, the host cell is genetically modified, comprising a polynucleotide integrated at a neutral site in the *S. spinosa* genome. The invention also provides a the use of a genetically modified host cell comprising a polynucleotide integrated therein, wherein the polynucleotide is capable of disrupting expression of a native gene encoding a spinosyn biosynthetic enzyme. In embodiments, the host cell is genetically modified to comprise a polynucleotide integrated at a neutral site, as defined herein.

Also provided is a method for improving spinosad production in a *S*. *spinosa* host cell or improving the fermentation process of a *S*. *Spinosa* strain, the method comprising integrating into an *S. spinosa* genome, an obscurin polyketide synthase gene cluster as a neutral site for integration and expression of target genes of interest. In an embodiment, the method further comprises integrating a polynucleotide at the heterologous obscurin polyketide synthase locus, preferably wherein the obscurin PKS gene *obsA* is the site of integration. Also provided is a genetically modified host cell comprising a heterologous obscurin polyketide synthase locus. Also provided is the use of a genetically modified host cell for stable expression.

Also provided is an expression construct comprising an obscurin polyketide synthase gene cluster, preferably wherein the expression construct is suitable for integration into a host cell genome. Also provided is a genetically modified host cell comprising an expression construct comprising an obscurin polyketide synthase gene cluster, preferably wherein the expression construct is suitable for integration into a host cell genome.

Also provided is a method for integrating a gene into chromosomal DNA of a *S. spinosa* host cell, the method comprising the steps of:
a) creating a phage library to identify a genomic locus;
b) cloning two genomic DNA fragments from the genomic locus, wherein one genomic fragment is cloned upstream of a gene to be integrated of a polynucleotide, and the second genomic fragment is cloned downstream of a gene to be integrated of said polynucleotide, and the resulting ligated DNA fragment becomes an integration plasmid;
c) introducing the plasmid of step (b) into a strain of *S. spinosa* by a transformation method;
d) obtaining transconjugants; and,
e) screening said transconjugants for presence of said polynucleotide.

In embodiments, the polynucleotide is as defined herein. In an embodiment, a genomic fragment may be an internal fragment as defined herein. In an embodiment, it may be a fragment of the obscurin polyketide synthase cluster, as defined herein.

Also provided is a genetically modified *S. spinosa* host cell, wherein a gene has been added, substituted or deleted by integrating a gene into the chromosomal DNA according to the method of the invention, wherein the gene which has been substituted is not identical to a native *S. spinosa* gene. Also provided is a genetically modified *S. spinosa* host cell, wherein a gene has been added, substituted or deleted by integrating a gene into the chromosomal DNA according to the method as defined of the invention, wherein the gene which has been added is identical to a native *S. spinosa* gene. In an embodiment, the gene is integrated into the chromosomal DNA at a neutral site, preferably wherein the neutral site is the obscurin polyketide synthase gene cluster, preferably the *obs*A gene.

Also provided is a method of preparing a *S*. *spinosa* strain in which a specific number of copies of a chosen gene or of a chosen DNA molecule are integrated into a *S. spinosa* genome, wherein said method comprises:
a) cloning said chosen gene or said chosen DNA molecule into a mobile element, wherein there is at least one marker gene located within the mobile element;
b) integrating said mobile element containing said chosen gene or said chosen DNA molecule into the chromosome or episome of said *S. spinosa* strain;
c) complementing said mobile element with a functional gene;
d) expressing said functional mobile element gene;
e) selecting said strain on a medium that is selectable for said marker gene in a manner such that a strain containing a specific number of copies of said chosen gene or chosen DNA molecule is obtained; and
f) recovering the strain thereby selected.

In an embodiment, the chromosome is contained on a plasmid and is introduced by transformation. In an embodiment, the plasmid includes a genomic locus used for integrating a polynucleotide. In an embodiment, the genomic locus used for integration of a polynucleotide is an obscurin polyketide synthase locus, preferably wherein the obscurin PKS gene obsA is the site of integration. In an embodiment, the mobile element is a genetic construct.

Whilst specific reference is made in the present application to *S. spinosa,* it is envisaged that the present invention in all aspects and embodiments is applicable to any spinosyn producing species.

In embodiments, transformation of host cells by methods other than genomic integration of a polynucleotide may be appropriate. For example, where additional polynucleotides are provided in the cell.

### BRIEF DESCRIPTION OF THE DRAWINGS

Preferred embodiments of the invention are described with reference to the drawings, in which:
FIG. 1 depicts mapping of the end sequence of the cosmid clones onto the obscurin gene cluster. The overlapping cosmid clones are shown. Solid bars indicate the actual size of the inserts in cosmid clone 1E3 and cosmid clone 2N14 relative to the obscurin gene cluster. The dotted lines indicate that only one end of the cosmid is within the obscurin gene cluster.
FIG. 2 depicts plasmid pIJ773, which contains the apramycin resistance expression cassette (labeled as aac(3) IV).

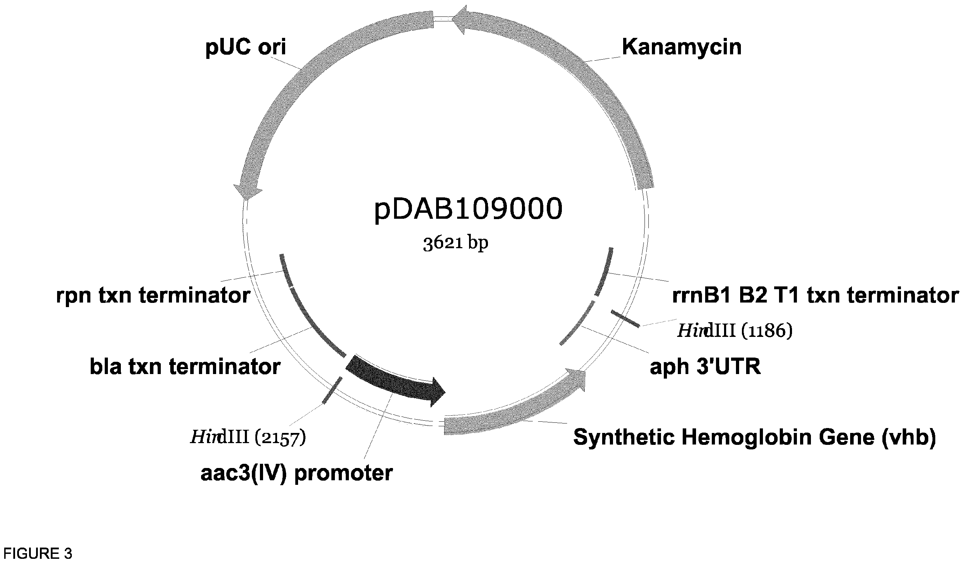

FIG. 3 depicts plasmid pDAB109000 containing the 977 bp synthetic DNA fragment with pJ201.

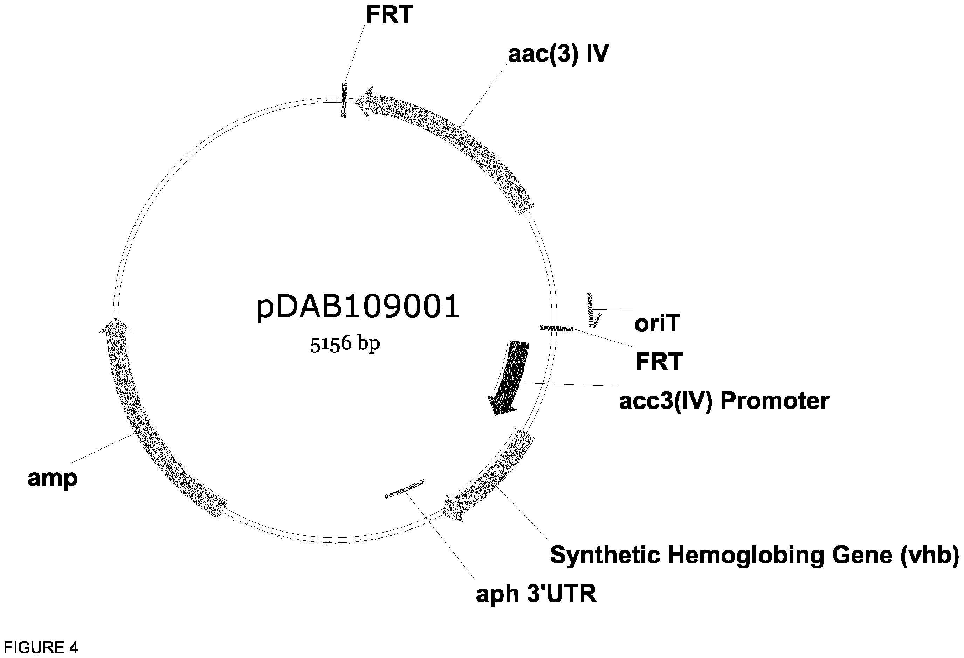

FIG. 4 depicts plasmid pDAB109001 which resulted from cloning of the hemoglobin gene expression cassette into pIJ773.

### DETAILED DESCRIPTION OF THE INVENTION

Molecular tools useful for metabolic engineering for spinosyn strain improvement include the identification and validation of neutral sites in the *S*. *spinosa* genome where gene expression cassettes can be introduced. Neutral sites are defined as DNA regions in the genome, which have little to no negative impact on the primary metabolic activities of *S. spinosa,* spinosyn production and optionally other desired characteristics. The identified neutral sites are intended for stable integration and optional expression of target polynucleotides either native or heterologous to *S. spinosa* which by way of example can include i) introducing beneficial characteristics to the existing spinosyn production strains, preferably a characteristic which is not exhibited by the same cell type which has not been genetically modified according to the invention, such as expression of a heterologous hemoglobin gene; ii) improving specific characteristics of the existing spinosyn production strains (e.g. improving the bioconversion of pseudoaglycones to spinosyns); iii) introducing multiple copies of the same polynucleotide(s) at one or more neutral sites, for example to maximize the benefit of the target polynucleotide(s) and to ensure the stability of the engineered recombinant strains; or iv) introducing multiple target polynucleotides to simultaneously de-bottleneck (e.g. overcome or ameliorate) two or more rate-limiting steps controlling spinosyn biosynthesis and production. The genes responsible for secondary metabolic pathways in *S. spinosa,* non-essential for the primary metabolic activities of the organism, represent an attractive source for neutral sites. Of these, the non-spinosyn polyketide synthase gene clusters are of particular interest since disruption of the gene clusters may result in the elimination of potential competing pathways for the common acyl-CoA precursors and cofactors essential for spinosyn biosynthesis and production. This may create synergy between the benefits of the target genes and increased availability of the precursors.

There are many different uses for integrated genes within the chromosome of *Saccharopolyspora spinosa.* The cloned genes can be used to improve yields of spinosyns and to produce new spinosyns. Improved yields can be obtained by integrating into the genome of a particular strain a duplicate copy of the gene to overcome an enzyme that is rate limiting in that strain. In some cases, where the biosynthetic pathway is blocked in a particular mutant strain due to lack of a required enzyme, production of the desired spinosyns can be restored by integrating a copy of the required gene. In other cases where a biosynthetic pathway is disrupted, a different precursor strain can be created.

The following definitions are used herein and should be referred to for interpretation of the claims and the specification.

As used herein, the indefinite articles "a" and "an" preceding an element or component of the invention are intended to be nonrestrictive regarding the number of instances (i.e., occurrences) of the element or component. Therefore "a" or "an" should be read to include one or at least one, and the singular word form of the element or component also includes the plural unless the number is obviously meant to be singular.

As used herein, the terms "comprising" and "including" mean the presence of the stated features, integers, steps, or components as referred to in the claims, but that it does not preclude the presence or addition of one or more other features, integers, steps, components or groups thereof. This means a composition, a mixture, a process, a method, an article, or an apparatus that "comprises" or "includes" a list of elements is not limited to only those elements but may include others not expressly listed or inherent to it. As used herein, "or" refers to an inclusive and an exclusive "or". For example, a condition A or B is satisfied by any one of the following: A is true (or present) and B is false (or not present), A is false (or not present) and B is true (or present), and both A and B are true (or present).

As used herein, the term "about" refers to modifying the quantity of an ingredient or reactant of the invention or employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities.

As used herein, the term "invention" or "present invention" is a non-limiting term and is intended to encompass all possible variations as described in the specification and recited in the claims.

As used herein, the terms "polypeptide" and "peptide" will be used interchangeably to refer to a polymer of two or more amino acids joined together by a peptide bond. In one aspect, this term also includes post expression modifications of the polypeptide, for example, glycosylations, acetylations, phosphorylations and the like. Included within the definition are, for example, peptides containing one or more analogues of an amino acid or labeled amino acids and peptidomimetics. The peptides may comprise L-amino acids. Reference herein to "polypeptide", "peptide" and "protein" include fragments, derivatives and variants thereof.

As used herein, the terms "peptide of interest", "POI", "gene product", "target gene product", and "target coding region gene product" refer to the desired peptide/protein product encoded by the expressed gene. The peptide of interest may include any peptide/protein product including, but not limited to proteins, fusion proteins, enzymes, peptides, polypeptides, and oligopeptides. The peptide of interest ranges in size from 2 to 398 amino acids in length. The desired protein/peptide product may be a heterologous peptide/protein, expressed by a foreign (i.e. non-native) gene.

As used herein, the term "genetic construct" refers to a series of contiguous nucleic acids useful for modulating the genotype or phenotype of an organism. Non-limiting examples of genetic constructs include but are not limited to a nucleic acid molecule, and open reading frame, a gene, an expression cassette, a vector, a plasmid and the like.

As used herein, the term "endogenous gene" refers to a native gene in its natural location in the genome of an organism.

As used herein, a "foreign gene" refers to a gene not normally found in the host organism, but that is introduced into the host organism by gene transfer. Foreign genes can comprise native genes inserted into a non-native organism, or chimeric genes. A foreign gene may also be referred to as a non-native gene. A foreign gene is preferably codon optimized for expression in a host cell, preferably where the host cell is *S. spinosa.* Suitable optimization for expression in *S. spinosa* is provided in Table 2.

Reference herein to a "gene" includes, for the purpose of the invention, any coding sequence or control sequence, or fragments thereof. A gene may include any combination of coding sequence and control sequence, or fragments thereof. Thus, a "gene" as referred to herein may be all or part of a native gene. A polynucleotide sequence as referred to herein may be used interchangeably with the term "gene", or may include any coding sequence, non-coding sequence or control sequence, fragments thereof, and combinations thereof.

As used herein, the term "heterologous" with respect to sequence within a particular organism/genome indicates that the sequence originates from a foreign species, or, if from the same species, is substantially modified from its native form in composition and/or genomic locus and/or copy number by deliberate human intervention. Thus, where the gene is a native gene, it may be heterologous if mutated, replicated (i.e. increased in copy number) or moved (i.e. its location within the cell). Thus, for example, heterologous gene expression refers to the process of expressing a gene from one organism/genome by placing it into the genome of a different organism/genome.

As used herein, the term "recombinant" refers to an artificial combination of two otherwise separated segments of sequence, e.g., by chemical synthesis or by the manipulation of isolated segments of nucleic acids by genetic engineering techniques. "Recombinant" also includes reference to a cell or vector, that has been modified by the introduction of a heterologous nucleic acid or a cell derived from a cell so modified, but does not encompass the alteration of the cell or vector by naturally occurring events (e.g., spontaneous mutation, natural transformation, natural transduction, natural transposition) such as those occurring without deliberate human intervention. A recombinant host cell of the present invention will be distinguishable from a naturally occurring cell of the same species by virtue of the presence of foreign DNA therein (e.g. selection markers, vectors, non-native genes), or by virtue of an altered genetic structure (e.g. removal or disruption of native sequences, changes in orientation of native sequences, altered promoters or other control sequences, altered copy numbers, altered location of native sequences). In addition to the above, a recombinant host cell of the invention will preferably show improved beneficial metabolic characteristics such as improved spinosyn production compared to a non-modified cell of the same type. With reference to cell, recombinant may be used interchangeably with the term "genetically modified".

A "strain" as referred to herein is a population of cells which are descended from a single organism or which are a substantially pure culture isolate. In an embodiment, a strain may comprise at least 50% 60%, 70%, 75%, 80%, 85%, 90%, 95% genetically modified host cells, as defined herein.

The term "genetically engineered" or "genetically altered" means the scientific alteration of the structure of genetic material in a living organism. It involves the production and use of recombinant DNA. More in particular it is used to delineate the genetically engineered or modified organism from the naturally occurring organism. Genetic engineering may be done by a number of techniques known in the art, such as e.g. gene replacement, gene amplification, gene disruption, transfection, transformation using plasmids, viruses, or other vectors. A genetically modified organism, e.g. genetically modified microorganism, is also often referred to as a recombinant organism, e.g. recombinant microorganism.

As used herein, the term "disrupted" or "disruption" refers to a gene that has been manipulated or modified through genetic engineering or through natural causes that change the activity of a gene. Such gene activity may be increased or decreased, compared to non-disrupted gene activity. Additionally, such disruption may alter or abolish protein expression and/or function. Disruption causing a decrease in gene activity may be a reduction in the copy number of a gene, thus causing underexpression of the gene. A gene is said to be "underexpressed" if the level of transcription of said gene is reduced in comparison to a non-disrupted gene (e.g. the wild type gene). This may be measured by for instance Northern blot analysis quantifying the amount of mRNA as an indication for gene expression. As used herein, a gene is underexpressed if the amount of generated mRNA is decreased by at least 1%, 2%, 5% 10%, 25%, 50%, 75%, 100%, 200% or even more than 500%, compared to the amount of mRNA generated from a non-disrupted gene (e.g. wild-type gene). Alternatively, disruption may include expression of the gene from a weak control sequence such as a promoter, compared to the native promoter. In another embodiment, the promoter, regulatory region and/or the ribosome binding site upstream of the gene can be altered to achieve the reduced expression. In another embodiment, the expression may also be reduced by decreasing the relative half-life of the messenger RNA. In another embodiment, the activity of a polypeptide itself may be decreased, for example by employing one or more mutations in the polypeptide amino acid sequence, which decrease the activity. For example, altering the affinity of the polypeptide for its corresponding substrate may result in reduced activity. Likewise, the relative half-life of the polypeptide may be decreased. In either scenario, that being reduced gene expression or reduced activity, the reduction may be achieved by altering the composition of the cell culture media and/or methods used for culturing. "Reduced expression" as used herein means a decrease of at least 5%, 10%, 25%, 50%, 75%, 100%, 200% or even more than 500%, compared to the amount of generated mRNA or protein compared to the non-disrupted gene (e.g. a wild-type gene). "Reduced activity" as used herein means a decrease of at least 5%, 10%, 25%, 50%, 75%, 100%, 200% or even more than 500%, compared to the activity or concentration of the protein, polynucleotide, or genebefore disruption. The activity of a protein may also be reduced by contacting the protein with a specific or general inhibitor of its activity. The terms "reduced activity", "decreased or abolished activity" are used interchangeably herein.

In another embodiment, disruption may increase the activity or expression of a gene. A control sequence such as the promoter, regulatory region and/or the ribosome binding site upstream of the gene can be altered to achieve increased expression. In another embodiment, increased expression may be achieved by increasing the copy number of the gene. In an embodiment, overexpression may also be achieved by increasing the relative half-life of the messenger RNA. In another embodiment, the activity of the polypeptide itself may be increased by employing one or more mutations in the polypeptide amino acid sequence, which increased the activity. For example, altering the affinity of the polypeptide for its corresponding substrate may result in increased activity. Likewise, the relative half-life of the polypeptide may be increased. In either scenario, that being gene overexpression or increased activity, the increase may be achieved by altering the composition of the cell culture media and/or methods used for culturing. "Overexpression " or "increased activity" as used herein means an increase of at least 5%, 10%, 25%, 50%, 75%, 100%, 200% or even more than 500%, compared to a wild-type protein, polynucleotide, gene; or the activity and/or the concentration of the protein present before the disruption to increase expression and/or activity. The activity of a protein may also be increased by contacting the protein with a specific or general inhibitor of its activity. The terms "Overexpression" and "increased activity" may be used interchangeably.

"Alter" with reference to changing expression or activity may include mutating, substituting, deleting or adding a gene, control sequence or polynucleotide sequence, or fragment thereof.

Herein, enhanced or increased spinosyn production means an increase in the amount one or more native spinosyns, or the production of one or more novel spinosyns, compared to the spinosyn production by a host cell of the same type when grown under the same conditions (for example shake flask fermentation) over the same time period. Preferably, the methods and host cells of the present invention provide for an increase in native spinosyn titer of at least 4%, 4.6%, 5%, 6%, 7%, 8%, 9%, 10%, 15%, 20%, 30%, 40%, 50%, 60%, 70%, 80%, 90% or at least 100%.

Expression "control sequences" refers collectively to promoter sequences, ribosome binding sites, transcription termination sequences, upstream regulatory domains, enhancers, and the like, which collectively provide for the transcription and translation of a coding sequence in a host cell. Any one or more of the above mentioned "control sequences" selected from the group mentioned above may be provided in an expression cassette. Not all of these control sequences need always be present in a recombinant vector so long as the desired gene is capable of being transcribed and translated.

Herein, reference to chromosomal DNA or the host cell genome may be used interchangeably.

"Recombination" refers to the reassortment of sections of DNA or RNA sequences between two DNA or RNA molecules. "Homologous recombination" occurs between two DNA molecules which hybridize by virtue of homologous or complementary nucleotide sequences present in each DNA molecule.

Reference herein to a "spinosyn enhancing gene" includes any polynucleotide sequence which, when provided in a *S*. *spinosa* host cell, has the effect of increasing expression or activity of a native spinosyn, or enabling the expression of a novel spinosyn or a derivative of a native spinosyn. A spinosyn enhancing gene may be a control sequence or a coding sequence, or both, or fragments thereof. Also included are variants and derivatives of native spinosyn enhancing genes. In an embodiment, a spinosyn enhancing gene can encode for an oxygen-binding protein. Furthermore, the oxygen-binding protein can be any protein which binds oxygen, particularly those which bind oxygen reversibly such as the globins. A spinosyn enhancing gene may be a gene encoding a spinosyn biosynthetic enzyme.

"Oxygen-binding proteins" that may be used in the invention include, but are not limited to, *Vitreoscilla* hemoglobin (VHb), *Alcaligenes eutrophus* flavohemoprotein, horse heart myoglobin, *E. coli* hemoprotein, *B. subtilis* hemoprotein, yeast flavohemoglobin, soybean leghemoglobin, lupin leghemoglobin, and sperm whale myoglobin. As noted above, the oxygen-binding protein may also be one that is endogenous to the spinosyn-producing organism. Alternatively, the oxygen binding protein may be heterologous to the spinosyn producing organism.

A "neutral site" for integration of a polynucleotide is one which encodes a gene product which is not involved in a primary metabolic pathway, and preferably is not required for spinosyn production. A neutral site may be one which encodes a gene which is not required for growth and/or division of the cell. Thus, if the gene at the neutral site is disrupted, a genetically modified host cell will exhibit primary metabolism and growth comparable to a host cell of the same type which has not been genetically modified, when maintained under the same conditions. A neutral site may be a site of a gene involved in a secondary metabolic pathway, for example the obscurin polyketide synthase (PKS) locus, preferably resulting in the inactivation of a native gene, *obsA.* The sequence of a fragment of the *obs*A gene is provided herein as SEQ ID NO 8. Where the neutral site is a gene, disruption may be to a control element of the gene or the coding sequence of the gene, or both. Alternatively, a neutral site may be a site which is not a gene.

A primary metabolic pathway includes those pathways essential for cell survival, for example glycolysis, the pentose phosphate pathway, the TCA cycle and amino acid biosynthesis. A secondary metabolic pathway is one which one is involved in the production of a secondary metabolite, i.e. one which is not required for survival of the individual cell.

The terms "stringent conditions" or "hybridization under stringent conditions" refers to conditions under which a probe will hybridize preferentially to its target subsequence, and to a lesser extent to, or not at all to, other sequences. "Stringent hybridization" and "stringent hybridization wash conditions" in the context of nucleic acid hybridization experiments such as Southern and northern hybridizations are sequence dependent, and are different under different environmental parameters. An extensive guide to the hybridization of nucleic acids is found in Tijssen (1993) Laboratory Techniques in Biochemistry and Molecular Biology--Hybridization with Nucleic Acid Probes part I chapter 2 Overview of principles of hybridization and the strategy of nucleic acid probe assays, Elsevier, New York. Generally, highly stringent hybridization and wash conditions are selected to be about 5°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Very stringent conditions are selected to be equal to the Tₘ for a particular probe.

An example of stringent hybridization conditions for hybridization of complementary nucleic acids, which have more than 100 complementary residues on a filter in a Southern or Northern blot is 50% formamide with 1 mg of heparin at 42°C, with the hybridization being carried out overnight. An example of highly stringent wash conditions is 0.15 M NaCl at 72°C for about 15 minutes. An example of stringent wash conditions is a 0.2xSSC wash at 65°C for 15 minutes (see, Sambrook et al., (1989) Molecular Cloning--A Laboratory Manual (2nd ed.) Vol. 1-3, Cold Spring Harbor Laboratory, Cold Spring Harbor Press, NY, for a description of SSC buffer). Often, a high stringency wash is preceded by a low stringency wash to remove background probe signal. An example medium stringency wash for a duplex of, e.g., more than 100 nucleotides, is 1xSSC at 45°C for 15 minutes. An example low stringency wash for a duplex of, e.g., more than 100 nucleotides, is 4-6xSSC at 40° C for 15 minutes. In general, a signal to noise ratio of 2x (or higher) than that observed for an unrelated probe in the particular hybridization assay indicates detection of a specific hybridization. Nucleic acids, which do not hybridize to each other under stringent conditions, are still substantially identical if the polypeptides which they encode are substantially identical. This occurs, e.g., when a copy of a nucleic acid is created using the maximum codon degeneracy permitted by the genetic code.

The invention also relates to an isolated polynucleotide hybridizable under stringent conditions, preferably under highly stringent conditions, to a polynucleotide as of the present invention.

As used herein, the term "hybridizing" is intended to describe conditions for hybridization and washing under which nucleotide sequences at least about 50%, at least about 60%, at least about 70%, more preferably at least about 80%, even more preferably at least about 85% to 90%, most preferably at least 95% homologous to each other typically remain hybridized to each other.

In one embodiment, a nucleic acid of the invention is at least 40%, 45%, 50%, 55%, 60%, 65%, 70%, 75%, 80%, 85%, 90%, 91%, 92%, 93%, 94%, 95%, 96%, 97%, 98%, 99%, or more homologous to a nucleic acid sequence shown in this application or the complement thereof.

Another non-limiting example of stringent hybridization conditions are hybridization in 6x sodium chloride/sodium citrate (SSC) at about 45°C, followed by one or more washes in 1xSSC, 0.1% SDS at 50°C, preferably at 55°C more preferably at 60°C and even more preferably at 65°C.

Highly stringent conditions can include incubations at 42°C for a period of several days, such as 2-4 days, using a labeled DNA probe, such as a digoxigenin (DIG)-labeled DNA probe, followed by one or more washes in 2xSSC, 0.1% SDS at room temperature and one or more washes in 0.5xSSC, 0.1% SDS or 0.1xSSC, 0.1% SDS at 65-68°C. In particular, highly stringent conditions include, for example, 2 h to 4 days incubation at 42°C using a DIG-labeled DNA probe (prepared by e.g. using a DIG labeling system; Roche Diagnostics GmbH, 68298 Mannheim, Germany) in a solution such as DigEasyHyb solution (Roche Diagnostics GmbH) with or without 100 µg/ml salmon sperm DNA, or a solution comprising 50% formamide, 5xSSC (150 mM NaCl, 15 mM trisodium citrate), 0.02% sodium dodecyl sulfate, 0.1% N-lauroylsarcosine, and 2% blocking reagent (Roche Diagnostics GmbH), followed by washing the filters twice for 5 to 15 minutes in 2xSSC and 0.1% SDS at room temperature and then washing twice for 15-30 minutes in 0.5xSSC and 0.1% SDS or 0.1xSSC and 0.1% SDS at 65-68°C.

In some embodiments an isolated nucleic acid molecule of the invention that hybridizes under highly stringent conditions to a nucleotide sequence of the invention can correspond to a naturally-occurring nucleic acid molecule. As used herein, a "naturally-occurring" nucleic acid molecule refers to an RNA or DNA molecule having a nucleotide sequence that occurs in nature (e.g., encodes a natural protein).

A skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N.Y.; and Ausubel et al., (eds.), 1995, Current Protocols in Molecular Biology, (John Wiley & Sons, N.Y.).

The present invention includes within its scope a method of improving spinosyn production in an *S. spinosa* host cell, comprising integrating a polynucleotide into a neutral site of chromosomal DNA of an *S. spinosa* cell, wherein the polynucleotide comprises a gene encoding a spinosyn biosynthetic enzyme. The present invention also provides a genetically modified *S. spinosa* host cell comprising a polynucleotide integrated into a neutral site of the genome, wherein the polynucleotide comprises a spinosyn enhancing gene and/or a gene encoding a spinosyn biosynthetic enzyme. The present invention also relates to the use of a *S*. *spinosa* host cell comprising a polynucleotide integrated into a neutral site of the genome, in the production of a spinosyn, wherein the polynucleotide comprises a spinosyn enhancing gene and/or a gene encoding a spinosyn biosynthetic enzyme. In an embodiment, a host cell of the invention shows improved spinosyn production, preferably A/D production or spinosyn J/L production in the shake flask fermentation.

A polynucleotide containing a gene for a spinosyn biosynthetic enzyme would enable provision or duplication of genes coding for rate limiting enzymes in the production of spinosyns. This could be used to increase yield in any circumstance when one of the encoded activities limited synthesis of the desired spinosyn. A yield increase in spinosyn A/D was observed when genes linked to the spinosyn polyketide synthase were duplicated by integrating a cosmid containing them into *S. spinosa* (Madduri et al., 2001). In another example, a yield increase of this type was achieved in fermentations of *Streptomyces fradiae* by duplicating the gene encoding a rate-limiting methyltransferase that converts macrocin to tylosin (Baltz et al., 1997).

In an embodiment, the present invention provides for the provision in a host cell of a gene encoding a spinosyn biosynthetic enzyme, in combination with the provision of a spinosyn enhancing gene, which in an embodiment may encode an oxygen binding protein. Thus, the invention provides for increasing spinosyn production by the heterologous expression of a spinosyn enhancing gene, and the co-expression of a heterologous spinosyn biosynthetic enzyme. Methods, host cells and uses of the invention relating to this embodiment are provided.

The present invention also includes within its scope a method of producing a novel spinosyn or a spinosyn intermediate or derivative, in an *S. spinosa* host cell, comprising integrating a polynucleotide into chromosomal DNA of a *S*. *spinosa* host cell, wherein the polynucleotide is capable of disrupting expression of a native gene encoding a spinosyn biosynthetic enzyme. In an embodiment, the polynucleotide comprises an internal fragment of a native gene encoding a spinosyn biosynthetic enzyme. The present invention also provides a genetically modified *S. spinosa* host cell comprising a polynucleotide integrated therein, wherein the polynucleotide is capable of disrupting expression of a native gene encoding a spinosyn biosynthetic enzyme. In an embodiment, the polynucleotide comprises an internal fragment of a native gene encoding a spinosyn biosynthetic enzyme. The present invention also relates to the use of a *S*. *spinosa* host cell comprising a polynucleotide integrated therein in the production of a spinosyn, wherein the polynucleotide is capable of disrupting expression of a native gene encoding a spinosyn biosynthetic enzyme. In an embodiment, the polynucleotide comprises an internal fragment of a native gene encoding a spinosyn biosynthetic enzyme. In an embodiment, a *S. spinosa* host cell of the invention shows improved spinosyn production, preferably A/D production or spinosyn J/L production in the shake flask fermentation. In an embodiment, a heterologous gene encoding a spinosyn biosynthetic enzyme may be provided in the genetically modified host cell. The heterologous gene may encode the same spinosyn biosynthetic enzyme or a different spinosyn biosynthetic enzyme. The heterologous gene may encode a native spinosyn biosynthetic enzyme or a mutant, derivative, fragment or variant thereof. Thus, the invention in an embodiment provides a method of producing a novel spinosyn or a spinosyn intermediate or derivative, in an *S. spinosa* host cell, comprising a) integrating a polynucleotide into chromosomal DNA of a *S*. *spinosa* host cell, wherein the polynucleotide is capable of disrupting expression of a native gene encoding a spinosyn biosynthetic enzyme; and b) integrating into a neutral site in the genome of the host cell a heterologous gene encoding a spinosyn biosynthetic enzyme and/or a heterologous spinosyn enhancing gene. Also provided is a genetically modified host cell comprising a polynucleotide integrated therein, wherein the polynucleotide is capable of disrupting expression of a native gene encoding a spinosyn biosynthetic enzyme, and further comprising a heterologous gene encoding a spinosyn biosynthetic enzyme and/or optionally a heterologous spinosyn enhancing gene. Also provided is the use of such as host cell in the improved production of a spinosyn.

Thus, specific intermediates (or their natural derivatives) could be synthesized by mutant strains of *S. spinosa* in which certain genes encoding enzymes for spinosyn biosynthesis have been disrupted. Such strains can be generated by integrating, via for example homologous recombination, a mutagenic sequence containing an internal fragment of the target gene. Upon integration, two incomplete copies of the biosynthetic gene are formed, thereby eliminating the enzymatic function it encoded. The substrate for this enzyme, or some natural derivative thereof, should accumulate upon fermentation of the mutant strain. Such a strategy was used effectively to generate a strain of *Saccharopolyspora erythraea* producing novel 6-deoxyerythromycin derivatives (Weber & McAlpine, 1992).

Such strains could be generated by swapping the target region, via double crossover homologous recombination, with a mutagenic plasmid containing the new fragment between non-mutated sequences, which flank the target region. The hybrid gene would produce protein with altered functions, either lacking an activity or performing a novel enzymatic transformation. A new derivative would accumulate upon fermentation of the mutant strain. Such a strategy was used to generate a strain of *Saccharopolyspora erythraea* producing a novel anhydroerythromycin derivative (Donadio et al., 1993).

Genes encoding spinosyn biosynthetic enzymes and related ORFs for use in the present invention were cloned and the DNA sequence of each was determined. The cloned genes and ORFs are designated hereinafter as *spnA, spnB, spnC, spnD, spnE, spnF, spnG, spnH, spnI, spnJ, spnK, spnL, spnM, spnN, spnO, spnP, spnQ, spnR, spnS,* ORFL15, ORFL16, ORFR1, ORFR2, *S. spinosa gtt, S. spinosa gdh, S. spinosa epi,* and *S. spinosa kre.* The sequences of the spinosyn biosynthetic genes are known in the art, and are defined in US2004/002343 (application No 10/329148) by bases 21111-28898, 28916-35374, 35419-44931, 44966-59752, 59803-76569, 20168-20995, 18541-19713, 17749-18501, 16556-17743, 14799-16418, 13592-14785, 12696-13547, 11530-12492, 10436-11434, 8967-10427, 7083-8450, 5363-6751, 4168-5325, 3416-4165, 2024-2791, 1135-1971, 76932-77528, and 77729-79984 of SEQ ID NO: 1, bases 334-1119 of SEQ ID NO:27, bases 88-1077 of SEQ ID NO 24, bases 226-834 of SEQ ID NO 31, and bases 1165-1992 of SEQ ID NO:24, respectively. Reference herein to a gene encoding a spinosyn biosynthetic enzyme includes any of the above mentioned genes and ORF's, as well as any additional genes and ORF's known to a person skilled in the art. Thus, the present invention comprises the use of one or more genes selected from the group mentioned above, or fragments, derivatives or variants thereof.

*Saccharapolyspora spinosa* produces a mixture of nine closely related compounds collectively called "spinosyns". Within the mixture, spinosyn A and D, known as spinsoad, are the major components and have activity against key insect targets. Spinosyn J and L, two of the minor components within the spinosyn mixture, are the precursors for spinetoram, another spinosyn insecticide.

Spinosad is an insecticide produced by Dow AgroSciences (Indianapolis, Ind.) that is comprised mainly of approximately 85% spinosyn A and approximately 15% spinosyn D. Spinosyn A and D are natural products produced by fermentation of *Saccharopolyspora spinosa,* as disclosed in U.S. Pat. No. 5,362,634. Spinosad is an active ingredient of several insecticidal formulations available commercially from Dow AgroSciences, including the TRACER™, SUCCESS™, SPINTOR™, and CONSERVE™ insect control products. For example, the TRACER product is comprised of about 44% to about 48% spinosad (w/v), or about 4 pounds of spinosad per gallon. Spinosyn compounds in granular and liquid formulations have established utility for the control of arachnids, nematodes, and insects, in particular Lepidoptera, Thysanoptera, and Diptera species. Spinosyn A and D is also referred to herein as Spinosyn A/D.

Spinetoram is a mixture of 5,6-dihydro-3'-ethoxy spinosyn J (major component) and 3'-ethoxy spinosyn L produced by Dow AgroSciences. The mixture can be prepared by ethoxylating a mixture of spinosyn J and spinosyn L, followed by hydrogenation. The 5,6 double bond of spinosyn J and its 3'-ethoxy is hydrogenated much more readily than that of spinosyn L and its 3'-ethoxy derivative, due to steric hindrance by the methyl group at C-5 in spinosyn L and its 3'-ethoxy derivative. See, U.S. Pat. No. 6,001,981. Spinosyn J and L is also referred to herein as Spinosyn J/L.

Conventional notation is used herein to describe polynucleotide sequences: the left-hand end of a single-stranded polynucleotide sequence is the 5'-end; the left-hand direction of a double-stranded polynucleotide sequence is referred to as the 5'-direction. The direction of 5' to 3' addition of nucleotides to nascent RNA transcripts is referred to as the transcription direction. The DNA strand having the same sequence as an mRNA is referred to as the "coding strand"; sequences on the DNA strand having the same sequence as an mRNA transcribed from that DNA and which are located 5' to the 5'-end of the RNA transcript are referred to as "upstream sequences"; sequences on the DNA strand having the same sequence as the RNA and which are 3' to the 3' end of the coding RNA transcript are referred to as "downstream sequences."

In certain embodiments for improving *S. spinosa* strain improvement, stable transformants of polynucleotide(s) were produced by integrating a gene expression cassette into the genome of *S. spinosa.* This was accomplished by integrating the gene via homologous recombination using a part of chromosomal DNA and an insertion element. Based on this recombination and as a result of application thereof, the *aac(3) IV* and *vhb* gene expression cassettes were separately integrated into the chromosome of *S. spinosa* at the obscurin polyketide synthase (PKS) locus resulting in the inactivation of a native gene, *obsA.*

Thus, the present invention provides a method of stably expressing a gene expression cassette in *S. spinosa,* comprising:
a) identifying and validating a neutral site in the *S. spinosa* genome, wherein a neutral site is one which can be disrupted without negatively impacting spinosyn production, *S. spinosa* growth or a desired metabolic characteristic;
b) introducing at said neutral site, a polynucleotide. Preferably, the polynucleotide is expressed stably over subsequent generations. In an embodiment, the polynucleotide comprises a gene expression cassette.

Preferably, a gene expression cassette used in the present invention comprises a fragment of a sequence of the obscurin polyketide synthase (PKS) locus. Preferably, a gene expression cassette comprises two or more separate fragments of said locus, preferably flanking a polynucleotide sequence to be inserted into a host cell genome. Preferably, any fragment may recombine with native gene, *obsA,* resulting in its inactivation.

Other embodiments of the present invention can include integrating a polynucleotide into the *S. spinosa* genome without negatively impacting spinosyn production, growth or other desired metabolic characteristics.

Additional embodiments of the present invention can include integrating a polynucleotide containing a gene expression cassette into the *S. spinosa* genome, the expression of which results in increased spinosyn production. It has been demonstrated in this application that the over expression of *VHb,* in *S. spinosa* results in increased spinosyn production. Expression of the oxygen carrier protein, VHb, produces elevated levels of the homodimeric hemoglobin under hypoxic growth conditions (Zhang et al., 2007). Among hemoglobins, *VHb* has an average oxygen association rate constant and a rather high oxygen dissociation rate constant (hundreds times higher than other hemoglobins). This suggests that *VHb* is able to release the bound oxygen more readily than all other hemoglobins (Zhang et al., 2007).

Expression of *VHb* in several *Actinomyces* strains led to increased antibiotic production including chlortetracycline, monensin, erythromycin and actinorhodin (Zhang et al., 2007; Magnolo et al., 1991). The recombinant *Streptomyces coelicolor* strain expressing *VHb* produced tenfold more actinorhodin than the wild-type strain under low DO levels (DO below 5% of air saturation) (Magnolo et al., 1991). In addition actinorhodin production by the *VHb*-expressing recombinant strain was less susceptible to aeration conditions. Although erythromycin production is in general not considered sensitive to DO levels as long as the DO levels are above the minimal levels required for growth, expression of VHb in an industrial erythromycin-producing strain significantly increased erythromycin titers to 7.25 g/L from 4.25 g/L while reduced biomass accumulation under fed-batch bioreactor fermentation conditions at scales between 10 - 15 liters (Brünker et al., 1997; 1998; Minas et al., 1998).

Oxygen-binding proteins that may be used in the invention include, but are not limited to, *Vitreoscilla* hemoglobin (VHb), *Alcaligenes eutrophus* flavohemoprotein, horse heart myoglobin, *E. coli* hemoprotein, *B. subtilis* hemoprotein, yeast flavohemoglobin, soybean leghemoglobin, lupin leghemoglobin, and sperm whale myoglobin. As noted above, the oxygen-binding protein may also be one that is endogenous to the spinosyn-producing organism. Alternatively, the oxygen binding protein may be heterologous to the spinosyn producing organism.

Genes encoding a large number of oxygen-binding proteins have been cloned and their sequence determined. For example, known polynucleotide sequences of globin proteins useful in the instant invention include but are not limited to those encoding a cyanobacterium myoglobin (Potts et al., 1992, Science 256:1690-1692), *Scapharca inaequivalvis* hemoglobin (Gambacurta et al., 1993, FEBS Lett. 330:90-94), *Aplysia limacina* myoglobin (Cutruzzola et al., 1996, Biochem. J. 314:83-90), Ascaris hemoglobin (Sherman et al., 1992, Proc. Natl. Acad. Sci. USA 89:11696-11700), *Pseudoterranova decipiens* nematode hemoglobin (Dixon et al., 1991, Natl. Acad. Sci. USA 88:5655-5659, and Dixon et al., 1992, J. Mol. Evol. 35:131-136). *Paramecium caudatum* hemoglobin (Yamauchi et al., 1992, Biochem. Biophvs. Res. Commun. 182:195-200), *Rhizobium meliloti* hemoprotein (David et al., 1988, Cell 54:671-683), and *Saccharomyces cerevisiae* (Shimada et al., 1989, J. Biochem. 105:417-422). Particularly suitable for use in the present invention are those oxygen- binding proteins which have relatively high k_{off} rates such as VHb (k_{off} 5600 s⁻¹; Orii and Webster, 1986, J. Biol. Chem. 261:3544-3547) or relatively low oxygen affinity such as horse heart myoglobin (K_{D} 0.79 µM; Wittenberg et al., 1985, in nitrogen fixation research progress. H.J. Evand et al., Eds. Martinus Nijhoff Publishers, Dordrecht, p. 354). Therefore, preferred oxygen binding proteins can be those proteins with a k_{off} rate for oxygen of greater than 10 s⁻¹, more preferred greater than 100 s⁻¹, or a K_{D} for oxygen of more than 0.5 µM, although it will be understood that oxygen-binding proteins with rate constants outside of these parameters will also be useful. As noted earlier, those preferred oxygen-binding proteins include globins such as hemoglobin, myoglobin, and leghemoglobins. The properties of many oxygen-binding proteins, including globins, are disclosed throughout the literature. Additionally, techniques for determining the oxygen-binding properties of a protein such as a globin are well known to one of skill in the art and can be performed without undue experimentation.

As noted earlier one such oxygen-binding protein for use in the instant invention, as described herein by way of working example, is *VHb.* The complete sequence of the *VHb* gene is described in U.S. Patent No. 5,049,493. Fragments, mutants, variants and derivatives of *VHb* which bind oxygen are also within the scope of the present invention. Also included are fragments, mutants, variants and derivatives of the polynucleotide sequence encoding VHb, which encode a protein which binds oxygen. Preferably, the present invention provides for the use of the VHb coding sequence of the expression vector disclosed in SEQ ID NO 1, or fragments, variants or derivatives thereof.

Embodiments of the present invention can also include integrating a polynucleotide into the *S. spinosa* genome at a neutral site, and the subsequent stacking of a second polynucleotide at the same location. Wherein, the neutral site within the *S. spinosa* is utilized as a preferred locus for introducing additional polynucleotides.

Other embodiments of the present invention can include integrating a polynucleotide into the *S. spinosa* genome at a neutral site, and the subsequent removal of a selectable marker from the integrated polynucleotide. In an embodiment, the polynucleotide comprises a gene expression cassette, which comprises a selectable marker. Any suitable method may be used. A preferred method used to remove the selectable marker expression cassette is a double crossing over method, an excision method using CRE-LOX, an excision method using FLP-FRT, or an excision method using the RED/ET RECOMBINATION® kit (Genebridges, Heidelberg, Germany), in addition to other excision methods known in the art. Thus, the method of the invention as defined herein may optionally comprise step c) removing a selectable marker expression cassette from the integrated gene expression cassette.

Additional embodiments of the present invention can include integrating a polynucleotide into the *S. spinosa* genome at a neutral site as an alternative to the use of extraneous replicating plasmids. Wherein, one or more extraneous replicating plasmids are incompatible due to the presence of similar origins or replication, incompatibility groups, redundant selectable marker, or other gene elements. Wherein, one or more extraneous replicating plasmids are not functional in S. *spinosa* due to the specificity of the *S. spinosa* restriction modification system. Wherein, one or more extraneous replicating plasmids are not available, functional or readily transformable within *S. spinosa.*

Other embodiments of the present invention can include methods for increasing the efficiency of homologous recombination in a prokaryotic cell. Methods relying upon homologous recombination mediated by introduced enzymes, such as lambda red 'recombineering' and analogous approaches are useful in a limited number of bacterial classes, particularly *Escherichia* (Datsenko and Wanner (2000) Proc Natl Acad Sci U S A. 97: 6640-5) and *Salmonella.* Methods relying upon site-specific recombination mediated by introduced enzymes, such as phage integrases, FLP/FRT or Cre/loxP may also be used, but are reliant on the presence of pre-existing sites within the target DNA (Wirth et al., (2007) Current Opinions in Biotechnology 18, 411-419). Alternative methods exploit viruses or mobile elements, or their components (e.g. phage, transposons or mobile introns).

However, methods relying upon host-mediated homologous recombination are by far the most commonly-used type of chromosomal DNA modifications. In a typical microbial application of host-mediated homologous recombination, a plasmid with a single region of sequence identity with the chromosome is integrated into the chromosome by single-crossover integration, sometimes referred to as Campbell-like integration'. After such an event, genes on the introduced plasmid are replicated as part of the chromosome, which may be more rapid than the plasmid replication. Accordingly, growth in medium with selection for a plasmid-borne selectable marker gene may provide a selective pressure for integration. Campbell-like integration can be used to inactivate a chromosomal gene by placing an internal fragment of a gene of interest on the plasmid, so that after integration, the chromosome will not contain a full-length copy of the gene. The chromosome of a Campbell-like integrant cell is not stable, because the integrated plasmid is flanked by the homologous sequences that directed the integration. A further homologous recombination event between these sequences leads to excision of the plasmid, and reversion of the chromosome to wild-type. For this reason, it may be necessary to maintain selection for the plasmid-borne selectable marker gene to maintain the integrant clone.

An improvement on the basic single-crossover integration method of chromosomal modification can include double crossover homologous recombination, also referred to as allelic exchange, which involves two recombination events. The desired modified allele is placed on a plasmid flanked by regions of homology to the regions flanking the target allele in the chromosome ('homology arms'). A first integration event can occur in either pair of homology arms, leading to integration of the plasmid into the chromosome in the same manner as Campbell-like integration. After the first crossover event, the chromosome contains two alternative sets of homologous sequences that can direct a second recombination event. If the same sequences that directed the first event recombine, the plasmid will be excised, and the cell will revert to wild-type. If the second recombination event is directed by the other homology arm, a plasmid will be excised, but the original chromosomal allele will have been exchanged for the modified allele introduced on the plasmid; the desired chromosomal modification will have been achieved. As with Campbell-like integration, the first recombination event is typically detected and integrants isolated using selective advantage conferred by integration of a plasmid-borne selectable marker gene.

"Functional polymorphism" as used herein refers to a change in the base pair sequence of a gene that produces a qualitative or quantitative change in the activity of the gene (e.g. expression of the gene) or in the activity and/or expression of protein encoded by that gene (e.g., a change in specificity of activity; a change in level of activity). The term "functional polymorphism" includes mutations, deletions and insertions.

In general, the step of detecting the polymorphism of interest may be carried out by collecting a biological sample containing DNA from the source, and then determining the presence or absence of DNA containing the polymorphism of interest in the biological sample.

Determining the presence or absence of DNA encoding a particular mutation may be carried out with an oligonucleotide probe labeled with a suitable detectable group, and/or by means of an amplification reaction such as a polymerase chain reaction or ligase chain reaction (the product of which amplification reaction may then be detected with a labeled oligonucleotide probe or a number of other techniques). Numerous different oligonucleotide probe assay formats are known which may be employed to carry out the present invention. See, e.g., U.S. Pat. No. 4,302,204 to Wahl et al.; U.S. Pat. No. 4,358,535 to Falkow et al.; U.S. Pat. No. 4,563,419 to Ranki et al.; and U.S. Pat. No. 4,994,373 to Stavrianopoulos et al.

Amplification of a selected, or target, nucleic acid sequence may be carried out by any suitable means. See generally, Kwoh et al., Am. Biotechnol. Lab. 8, 14-25 (1990). Examples of suitable amplification techniques include, but are not limited to, polymerase chain reaction, ligase chain reaction, strand displacement amplification (see generally G. Walker et al., Proc. Natl. Acad. Sci. USA 89, 392-396 (1992); G. Walker et al., Nucleic Acids Res. 20, 1691-1696 (1992)), transcription-based amplification (see D. Kwoh et al., Proc. Natl. Acad Sci. USA 86, 1173-1177 (1989)), self-sustained sequence replication (or "3SR") (see J. Guatelli et al., Proc. Natl. Acad. Sci. USA 87, 1874-1878 (1990)), the Qβ replicase system (see P. Lizardi et al., BioTechnology 6, 1197-1202 (1988)), nucleic acid sequence-based amplification (or "NASBA") (see R. Lewis, Genetic Engineering News 12 (9), 1 (1992)), the repair chain reaction (or "RCR") (see R. Lewis, supra), and boomerang DNA amplification (or "BDA") (see R. Lewis, supra). Polymerase chain reaction is generally preferred.

Polymerase chain reaction (PCR) may be carried out in accordance with known techniques. See, e.g., U.S. Pat. Nos. 4,683,195; 4,683,202; 4,800,159; and 4,965,188. In general, PCR involves, first, treating a nucleic acid sample (e.g., in the presence of a heat stable DNA polymerase) with one oligonucleotide primer for each strand of the specific sequence to be detected under hybridizing conditions so that an extension product of each primer is synthesized which is complementary to each nucleic acid strand, with the primers sufficiently complementary to each strand of the specific sequence to hybridize therewith so that the extension product synthesized from each primer, when it is separated from its complement, can serve as a template for synthesis of the extension product of the other primer, and then treating the sample under denaturing conditions to separate the primer extension products from their templates if the sequence or sequences to be detected are present. These steps are cyclically repeated until the desired degree of amplification is obtained. Detection of the amplified sequence may be carried out by adding to the reaction product an oligonucleotide probe capable of hybridizing to the reaction product (e.g., an oligonucleotide probe of the present invention), the probe carrying a detectable label, and then detecting the label in accordance with known techniques, or by direct visualization on a gel. Such probes may be from 5 to 500 nucleotides in length, preferably 5 to 250, more preferably 5 to 100 or 5 to 50 nucleic acids. When PCR conditions allow for amplification of all allelic types, the types can be distinguished by hybridization with an allelic specific probe, by restriction endonuclease digestion, by electrophoresis on denaturing gradient gels, or other techniques.

Ligase chain reaction (LCR) is also carried out in accordance with known techniques. See, e.g., R. Weiss, Science 254, 1292 (1991). In general, the reaction is carried out with two pairs of oligonucleotide probes: one pair binds to one strand of the sequence to be detected; the other pair binds to the other strand of the sequence to be detected. Each pair together completely overlaps the strand to which it corresponds. The reaction is carried out by, first, denaturing (e.g., separating) the strands of the sequence to be detected, then reacting the strands with the two pairs of oligonucleotide probes in the presence of a heat stable ligase so that each pair of oligonucleotide probes is ligated together, then separating the reaction product, and then cyclically repeating the process until the sequence has been amplified to the desired degree. Detection may then be carried out in like manner as described above with respect to PCR.

DNA amplification techniques such as the foregoing can involve the use of a probe, a pair of probes, or two pairs of probes which specifically bind to DNA containing the functional polymorphism, but do not bind to DNA that does not contain the functional polymorphism. Alternatively, the probe or pair of probes could bind to DNA that both does and does not contain the functional polymorphism, but produce or amplify a product (e.g., an elongation product) in which a detectable difference may be ascertained (e.g., a shorter product, where the functional polymorphism is a deletion mutation). Such probes can be generated in accordance with standard techniques from the known sequences of DNA in or associated with a gene linked to *obsA* or from sequences which can be generated from such genes in accordance with standard techniques.

It will be appreciated that the detecting steps described herein may be carried out directly or indirectly. Other means of indirectly determining allelic type include measuring polymorphic markers that are linked to the particular functional polymorphism, as has been demonstrated for the VNTR (variable number tandem repeats).

Molecular biology comprises a wide variety of techniques for the analysis of nucleic acid and protein sequences. Many of these techniques and procedures form the basis of clinical diagnostic assays and tests. These techniques include nucleic acid hybridization analysis, restriction enzyme analysis, genetic sequence analysis, and the separation and purification of nucleic acids and proteins (See, e.g., J. Sambrook, E. F. Fritsch, and T. Maniatis, Molecular Cloning: A Laboratory Manual, 2 Ed., Cold spring Harbor Laboratory Press, Cold Spring Harbor, N.Y., 1989).

Most of these techniques involve carrying out numerous operations (e.g., pipetting, centrifugation, and electrophoresis) on a large number of samples. They are often complex and time consuming, and generally require a high degree of accuracy. Many techniques are limited in their application by a lack of sensitivity, specificity, or reproducibility.

Nucleic acid hybridization analysis generally involves the detection of a very small number of specific target nucleic acids (DNA or RNA) with an excess of probe DNA, among a relatively large amount of complex non-target nucleic acids. A reduction in the complexity of the nucleic acid in a sample is helpful to the detection of low copy numbers (i.e. 10,000 to 100,000) of nucleic acid targets. DNA complexity reduction is achieved to some degree by amplification of target nucleic acid sequences. (See, M. A. Innis et al., PCR Protocols: A Guide to Methods and Applications, Academic Press, 1990, Spargo et al., 1996, Molecular & Cellular Probes, in regard to SDA amplification). This is because amplification of target nucleic acids results in an enormous number of target nucleic acid sequences relative to non-target sequences thereby improving the subsequent target hybridization step.

The hybridization step involves placing the prepared DNA sample in contact with a specific reporter probe at set optimal conditions for hybridization to occur between the target DNA sequence and probe. Hybridization may be performed in any one of a number of formats. For example, multiple sample nucleic acid hybridization analysis has been conducted in a variety of filter and solid support formats (See Beltz et al., Methods in Enzymology, Vol. 100, Part et al., Eds., Academic Press, New York, Chapter 19, pp. 266-308, 1985). One format, the so-called "dot blot" hybridization, involves the non-covalent attachment of target DNAs to a filter followed by the subsequent hybridization to a radioisotope labeled probe(s). "Dot blot" hybridization gained wide-spread use over the past two decades during which time many versions were developed (see Anderson and Young, in Nucleic Acid Hybridization--A Practical Approach, Hames and Higgins, Eds., IRL Press, Washington, D.C. Chapter 4, pp. 73-111, 1985). For example, the dot blot method has been developed for multiple analyses of genomic mutations (EPA 0228075 to Nanibhushan et al.) and for the detection of overlapping clones and the construction of genomic maps (U.S. Pat. No. 5,219,726 to Evans).

Additional techniques for carrying out multiple sample nucleic acid hybridization analysis include micro-formatted multiplex or matrix devices (e.g., DNA chips) (see M. Barinaga, 253 Science, pp. 1489, 1991; W. Bains, 10 Bio/Technology, pp. 757-758, 1992). These methods usually attach specific DNA sequences to very small specific areas of a solid support, such as micro-wells of a DNA chip. These hybridization formats are micro-scale versions of the conventional "dot blot" and "sandwich" hybridization systems.

The micro-formatted hybridization can be used to carry out "sequencing by hybridization" (SBH) (see M. Barinaga, 253 Science, pp. 1489, 1991; W. Bains, 10 Bio/Technology, pp. 757-758, 1992). SBH makes use of all possible n-nucleotide oligomers (n-mers) to identify n-mers in an unknown DNA sample, which are subsequently aligned by algorithm analysis to produce the DNA sequence (See, Drmanac U.S. Pat. No. 5,202,231).

There are two formats for carrying out SBH. The first format involves creating an array of all possible n-mers on a support, which is then hybridized with the target sequence. The second format involves attaching the target sequence to a support, which is sequentially probed with all possible n-mers. Southern, (United Kingdom Patent Application GB 8810400, 1988; E. M. Southern et al., 13 Genomics 1008, 1992), proposed using the first format to analyze or sequence DNA. Southern identified a known single point mutation using PCR amplified genomic DNA. Southern also described a method for synthesizing an array of oligonucleotides on a solid support for SBH. Drmanac et al., (260 Science 1649-1652, 1993), used a second format to sequence several short (116 bp) DNA sequences. Target DNAs were attached to membrane supports ("dot blot" format). Each filter was sequentially hybridized with 272 labeled 10-mer and 1-mer oligonucleotides. Wide ranges of stringency conditions were used to achieve specific hybridization for each n-mer probe. Washing times varied from 5 minutes to overnight using temperatures from 0°C to 16°C. Most probes required 3 hours of washing at 16°C. The filters had to be exposed from 2 to 18 hours in order to detect hybridization signals.

Generally, a variety of methods are available for detection and analysis of the hybridization events. Depending on the reporter group (fluorophore, enzyme, radioisotope, etc.) used to label the DNA probe, detection and analysis are carried out fluorimetrically, calorimetrically, or by autoradiography. By observing and measuring emitted radiation, such as fluorescent radiation or particle emission, information may be obtained about the hybridization events. Even when detection methods have very high intrinsic sensitivity, detection of hybridization events is difficult because of the background presence of non-specifically bound materials. Thus, detection of hybridization events is dependent upon how specific and sensitive hybridization can be made. Concerning genetic analysis, several methods have been developed that have attempted to increase specificity and sensitivity.

One form of genetic analysis is analysis centered on elucidation of single nucleic acid polymorphisms or ("SNPs"). Factors favoring the usage of SNPs are their high abundance in the human genome (especially compared to short tandem repeats, (STRs)), their frequent location within coding or regulatory regions of genes (which can affect protein structure or expression levels), and their stability when passed from one generation to the next (Landegren et al., Genome Research, Vol. 8, pp. 769-776, 1998).

A SNP is defined as any position in the genome that exists in two variants and the most common variant occurs less than 99% of the time. In order to use SNPs as widespread genetic markers, it is crucial to be able to genotype them easily, quickly, accurately, and cost-effectively. Numerous techniques are currently available for typing SNPs (for review, see Landegren et al., Genome Research, Vol. 8, pp. 769-776, (1998), all of which require target amplification. They include direct sequencing (Carothers et al., BioTechniques, Vol. 7, pp. 494-499, 1989), single-strand conformation polymorphism (Orita et al., Proc. Natl. Acad. Sci. USA, Vol. 86, pp. 2766-2770, 1989), allele-specific amplification (Newton et al., Nucleic Acids Research, Vol. 17, pp. 2503-2516, (1989), restriction digestion (Day and Humphries, Analytical Biochemistry, Vol. 222, pp. 389-395, 1994), and hybridization assays. In their most basic form, hybridization assays function by discriminating short oligonucleotide reporters against matched and mismatched targets. Many adaptations to the basic protocol have been developed. These include ligation chain reaction (Wu and Wallace, Gene, Vol. 76, pp. 245-254, 1989) and minisequencing (Syvanen et al., Genomics, Vol. 8, pp. 684-692, 1990). Other enhancements include the use of the 5'-nuclease activity of Taq DNA polymerase (Holland et al., Proc. Natl. Acad. Sci. USA, Vol. 88, pp. 7276-7280, 1991), molecular beacons (Tyagi and Kramer, Nature Biotechnology, Vol. 14, pp. 303-308, 1996), heat denaturation curves (Howell et al., Nature Biotechnology, Vol. 17, pp. 87-88, 1999) and DNA "chips" (Wang et al., Science, Vol. 280, pp. 1077-1082, 1998).

An additional phenomenon that can be used to distinguish SNPs is the nucleic acid interaction energies or base-stacking energies derived from the hybridization of multiple target specific probes to a single target. (See, R. Ornstein et al., "An Optimized Potential Function for the Calculation of Nucleic Acid Interaction Energies", Biopolymers, Vol.17, 2341-2360 (1978); J. Norberg and L. Nilsson, Biophysical Journal, Vol. 74, pp. 394-402, (1998); and J. Pieters et al., Nucleic Acids Research, Vol. 17, no. 12, pp. 4551-4565 (1989)). This base-stacking phenomenon is used in a unique format in the current invention to provide highly sensitive Tm differentials allowing the direct detection of SNPs in a nucleic acid sample.

Additional methods have been used to distinguish nucleic acid sequences in related organisms or to sequence DNA. For example, U.S. Pat. No. 5,030,557 by Hogan et al., disclosed that the secondary and tertiary structure of a single stranded target nucleic acid may be affected by binding "helper" oligonucleotides in addition to "probe" oligonucleotides causing a higher Tm to be exhibited between the probe and target nucleic acid. That application however was limited in its approach to using hybridization energies only for altering the secondary and tertiary structure of self-annealing RNA strands, which if left unaltered would tend to prevent the probe from hybridizing to the target.

With regard to DNA sequencing, K. Khrapko et al., Federation of European Biochemical Societies Letters, Vol. 256, no. 1,2, pp. 118-122 (1989), for example, disclosed that continuous stacking hybridization resulted in duplex stabilization. Additionally, J. Kieleczawa et al., Science, Vol. 258, pp. 1787-1791 (1992), disclosed the use of contiguous strings of hexamers to prime DNA synthesis wherein the contiguous strings appeared to stabilize priming. Likewise, L. Kotler et al., Proc. Natl. Acad. Sci. USA, Vol. 90, pp. 4241-4245, (1993) disclosed sequence specificity in the priming of DNA sequencing reactions by use of hexamer and pentamer oligonucleotide modules. Further, S. Parinov et al., Nucleic Acids Research, Vol. 24, no. 15, pp. 2998-3004, (1996), disclosed the use of base-stacking oligomers for DNA sequencing in association with passive DNA sequencing microchips. Moreover, G. Yershov et al., Proc. Natl. Acad. Sci. USA, Vol. 93, pp. 4913-4918 (1996), disclosed the application of base-stacking energies in SBH on a passive microchip. In Yershov's example, 10-mer DNA probes were anchored to the surface of the microchip and hybridized to target sequences in conjunction with additional short probes, the combination of which appeared to stabilize binding of the probes. In that format, short segments of nucleic acid sequence could be elucidated for DNA sequencing. Yershov further noted that in their system the destabilizing effect of mismatches was increased using shorter probes (e.g., 5-mers). Use of such short probes in DNA sequencing provided the ability to discern the presence of mismatches along the sequence being probed rather than just a single mismatch at one specified location of the probe/target hybridization complex. Use of longer probes (e.g., 8-mer, 10-mer, and 13-mer oligos) was less functional for such purposes.

An additional example of methodologies that have used base-stacking in the analysis of nucleic acids includes U.S. Pat. No. 5,770,365 by Lane et al., wherein is disclosed a method of capturing nucleic acid targets using a unimolecular capture probe having a single stranded loop and a double stranded region which acts in conjunction with a binding target to stabilize duplex formation by stacking energies.

The nucleotide sequence may be conveniently modified by site-directed mutagenesis in accordance with conventional methods. Alternatively, the nucleotide sequence may be prepared by chemical synthesis, including but not limited to, by using an oligonucleotide synthesizer, wherein oligonucleotides are designed based on the amino acid sequence of the desired polypeptide, and preferably selecting those codons that are favored in the host cell in which the recombinant polypeptide will be produced.

Novel spinosyns can also be produced by mutagenesis of the cloned genes, and substitution of the mutated genes for their unmutated counterparts in a spinosyn-producing organism. Preferably, substitution comprises a method of polynucleotide introduction is as described herein. Any suitable method of mutagenesis may be used, including for example mutagenesis of any spinosyn biosynthetic enzyme either directly or via mutagenesis of a gene encoding a spinosyn biosynthetic enzyme. The invention provides for mutagenesis of any spinosyn of *S. spinosa.* Preferably, mutagenesis may involve, for example: 1) deletion or inactivation of a ketoreductase, dehydratase or enoyl reductase (KR, DH, or ER) domain so that one or more of these functions is blocked and the strain produces a spinosyn having a lactone nucleus with a double bond, a hydroxyl group, or a keto group that is not present in the nucleus of spinosyn A (see Donadio et al., 1993); 2) replacement of an AT domain so that a different carboxylic acid is incorporated in the lactone nucleus (see Ruan et al., 1997); 3) addition of a KR, DH, or ER domain to an existing PKS module so that the strain produces a spinosyn having a lactone nucleus with a saturated bond, hydroxyl group, or double bond that is not present in the nucleus of spinosyn A; or 4) addition or subtraction of a complete PKS module so that the cyclic lactone nucleus has a greater or lesser number of carbon atoms. A hybrid PKS can be created by replacing the spinosyn PKS loading domain with heterologous PKS loading. See, e.g., US Patent No: 7,626,010. It has further been noted that spinosyns via modification of the sugars that are attached to the spinosyn lactone backbone can include modifications of the rhamnose and/or forosamine moiety or attachment of different deoxy sugars. The Salas group in Spain demonstrated that novel polyketide compounds can be produced by substituting the existing sugar molecule with different sugar molecules. Rodriguez et al., J. Mol. Microbiol Biotechnol. 2000 Jul;2(3):271-6. The examples that follow throughout the application help to illustrate the use of mutagenesis to produce a spinosyn with modified functionality.

Novel spinosyns can also be produced by mutagenesis of the cloned genes, and substitution of the mutated genes for their unmutated counterparts in a spinosyn-producing organism. Preferably, substitution comprises a method of polynucleotide introduction is as described herein. Any suitable method of mutagenesis may be used, including for example mutagenesis of any spinosyn biosynthetic enzyme either directly or via mutagenesis of a gene encoding a spinosyn biosynthetic enzyme. The invention provides for mutagenesis of any spinosyn of *S. spinosa.* Preferably, mutagenesis may involve, for example: 1) deletion or inactivation of a ketoreductase, dehydratase or enoyl reductase (KR, DH, or ER) domain so that one or more of these functions is blocked and the strain produces a spinosyn having a lactone nucleus with a double bond, a hydroxyl group, or a keto group that is not present in the nucleus of spinosyn A (see Donadio et al., 1993); 2) replacement of an AT domain so that a different carboxylic acid is incorporated in the lactone nucleus (see Ruan et al., 1997); 3) addition of a KR, DH, or ER domain to an existing PKS module so that the strain produces a spinosyn having a lactone nucleus with a saturated bond, hydroxyl group, or double bond that is not present in the nucleus of spinosyn A; or 4) addition or subtraction of a complete PKS module so that the cyclic lactone nucleus has a greater or lesser number of carbon atoms. A hybrid PKS can be created by replacing the spinosyn PKS loading domain with heterologous PKS loading. See, e.g., US Patent No: 7,626,010. It has further been noted that spinosyns via modification of the sugars that are attached to the spinosyn lactone backbone can include modifications of the rhamnose and/or forosamine moiety or attachment of different deoxy sugars. The Salas group in Spain demonstrated that novel polyketide compounds can be produced by substituting the existing sugar molecule with different sugar molecules. Rodriguez et al., J. Mol. Microbiol Biotechnol. 2000 Jul;2(3):271-6. The examples that follow throughout the application help to illustrate the use of mutagenesis to produce a spinosyn with modified functionality.

The DNA from the spinosyn gene cluster region can be used as a hybridization probe to identify homologous sequences. Thus, the DNA cloned here could be used to locate additional plasmids from the *Saccharopolyspora spinosa* gene libraries, which overlap the region described here but also contain previously uncloned DNA from adjacent regions in the genome of *Saccharopolyspora spinosa.* In addition, DNA from the region cloned here may be used to identify non-identical but similar sequences in other organisms. Hybridization probes are normally at least about 20 bases long and are labeled to permit detection.

Various types of mutagenesis can be used in the invention for a variety of purposes. They include, but are not limited to, site-directed, random point mutagenesis, homologous recombination, DNA shuffling or other recursive mutagenesis methods, chimeric construction, mutagenesis using uracil containing templates, oligonucleotide-directed mutagenesis, phosphorothioate-modified DNA mutagenesis, mutagenesis using gapped duplex DNA or the like, or any combination thereof. Additional suitable methods include point mismatch repair, mutagenesis using repair-deficient host strains, restriction-selection and restriction-purification, deletion mutagenesis, mutagenesis by total gene synthesis, double-strand break repair, and the like. Mutagenesis, including but not limited to, involving chimeric constructs, are also included in the present invention. In one embodiment, mutagenesis can be guided by known information of the naturally occurring molecule or altered or mutated naturally occurring molecule, including but not limited to, sequence, sequence comparisons, physical properties, crystal structure or the like.

The texts and examples found herein describe these procedures. Additional information is found in the following publications and references cited within: Ling et al., Approaches to DNA mutagenesis: an overview, Anal Biochem. 254(2): 157-178 (1997); Dale et al., Oligonucleotide-directed random mutagenesis using the phosphorothioate method, Methods Mol. Biol. 57:369-374 (1996); Smith, In vitro mutagenesis, Ann. Rev. Genet. 19:423-462(1985); Botstein & Shortle, Strategies and applications of in vitro mutagenesis, Science 229:1193-1201(1985); Carter, Site-directed mutagenesis, Biochem. J. 237:1-7 (1986); Kunkel, The efficiency of oligonucleotide directed mutagenesis, in Nucleic Acids & Molecular Biology (Eckstein, F. and Lilley, D. M. J. eds., Springer Verlag, Berlin) (1987); Kunkel, Rapid and efficient site-specific mutagenesis without phenotypic selection, Proc. Natl. Acad. Sci. USA 82:488-492 (1985); Kunkel et al., Rapid and efficient site-specific mutagenesis without phenotypic selection, Methods in Enzymol. 154, 367-382 (1987); Bass et al., Mutant Trp repressors with new DNA-binding specificities, Science 242:240-245 (1988); Methods in Enzymol. 100: 468-500 (1983); Methods in Enzymol. 154: 329-350 (1987); Zoller & Smith, Oligonucleotide-directed mutagenesis using M13-derived vectors: an efficient and general procedure for the production of point mutations in any DNA fragment, Nucleic Acids Res. 10:6487-6500 (1982); Zoller & Smith, Oligonucleotide-directed mutagenesis of DNA fragments cloned into M13 vectors, Methods in Enzymol. 100:468-500 (1983); Zoller & Smith, Oligonucleotide-directed mutagenesis: a simple method using two oligonucleotide primers and a single-stranded DNA template, Methods in Enzymol. 154:329-350 (1987); Taylor et al., The use of phosphorothioate-modified DNA in restriction enzyme reactions to prepare nicked DNA, Nucl. Acids Res. 13: 8749-8764 (1985); Taylor et al., The rapid generation of oligonucleotide-directed mutations at high frequency using phosphorothioate-modified DNA, Nucl. Acids Res. 13: 8765-8787 (1985); Nakamaye & Eckstein, Inhibition of restriction endonuclease Nci I cleavage by phosphorothioate groups and its application to oligonucleotide-directed mutagenesis, Nucl. Acids Res. 14: 9679-9698 (1986); Sayers et al., Y-T Exonucleases in phosphorothioate-based oligonucleotide-directed mutagenesis, Nucl. Acids Res. 16:791-802 (1988); Sayers et al., Strand specific cleavage of phosphorothioate-containing DNA by reaction with restriction endonucleases in the presence of ethidium bromide, (1988) Nucl. Acids Res. 16: 803-814; Kramer et al., The gapped duplex DNA approach to oligonucleotide-directed mutation construction, Nucl. Acids Res. 12: 9441-9456 (1984); Kramer & Fritz Oligonucleotide-directed construction of mutations via gapped duplex DNA, Methods in Enzymol. 154:350-367 (1987); Kramer et al., Improved enzymatic in vitro reactions in the gapped duplex DNA approach to oligonucleotide-directed construction of mutations, Nucl. Acids Res. 16: 7207 (1988); Fritz et al., Oligonucleotide-directed construction of mutations: a gapped duplex DNA procedure without enzymatic reactions in vitro, Nucl. Acids Res. 16: 6987-6999 (1988); Kramer et al., Point Mismatch Repair, Cell 38:879-887 (1984); Carter et al., Improved oligonucleotide site-directed mutagenesis using M13 vectors, Nucl. Acids Res. 13: 4431-4443 (1985); Carter, Improved oligonucleotide-directed mutagenesis using M13 vectors, Methods in Enzymol. 154: 382-403 (1987); Eghtedarzadeh & Henikoff, Use of oligonucleotides to generate large deletions, Nucl. Acids Res. 14: 5115 (1986); Wells et al., Importance of hydrogen-bond formation in stabilizing the transition state of subtilisin, Phil. Trans. R. Soc. Lond. A 317: 415-423 (1986); Nambiar et al., Total synthesis and cloning of a gene coding for the ribonuclease S protein, Science 223: 1299-1301 (1984); Sakamar and Khorana, Total synthesis and expression of a gene for the a-subunit of bovine rod outer segment guanine nucleotide-binding protein (transducin), Nucl. Acids Res. 14: 6361-6372 (1988); Wells et al., Cassette mutagenesis: an efficient method for generation of multiple mutations at defined sites, Gene 34:315-323 (1985); Grundstrom et al., Oligonucleotide-directed mutagenesis by microscale 'shot-gun' gene synthesis, Nucl. Acids Res. 13: 3305-3316 (1985); Mandecki, Oligonucleotide-directed double-strand break repair in plasmids of Escherichia coli: a method for site-specific mutagenesis, Proc. Natl. Acad. Sci. USA, 83:7177-7181 (1986); Arnold, Protein engineering for unusual environments, Current Opinion in Biotechnology 4:450-455 (1993); Sieber, et al., Nature Biotechnology, 19:456-460 (2001). W. P. C. Stemmer, Nature 370, 389-91 (1994); and, I. A. Lorimer, I. Pastan, Nucleic Acids Res. 23, 3067-8 (1995). Additional details on many of the above methods can be found in Methods in Enzymology Volume 154, which also describes useful controls for trouble-shooting problems with various mutagenesis methods.

Fragments of the peptides, polypeptides, proteins, polynucleotides and genes described herein may also be used in the invention. Fragments may comprise at least 20%, 30%, 40%, 50%, 60%, 70%, 75%, 80%, 85%, 90%, 91%, 92%, 93%, 94%, 95%, 96%, 97%, 98% or at least 99% or the length of the reference sequence (e.g. wild type or naturally occurring sequence). Preferred fragments are those which are functionally equivalent in at least one respect to the reference sequence.

The terms "homology" or "percent identity" are used interchangeably herein. For the purpose of this invention, it is defined here that in order to determine the percent identity of two amino acid sequences or of two nucleic acid sequences, the sequences are aligned for optimal comparison purposes (e.g., gaps may be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=number of identical positions/total number of positions (i.e., overlapping positionsx100). Preferably, the two sequences are the same length. In the present invention, a variant or derivative peptide, polypeptide, protein or polynucleotide or gene sequence is one which comprises one or more deletions, additions or substitutions, and may have at least 20%, 30%, 40%, 50%, 60%, 70%, 75%, 80%, 90%, 91%, 92%, 93%, 94%, 95%, 96%, 97%, 98%, or at least 99% percent sequence identity to the reference (e.g. wild type or naturally occurring) sequence. In a variant or derivative, the substitutions may be conservative substitutions, in which the amino acids or nucleic acids are replaced by amino acids or nucleic acids having similar properties such that the nature and activity of the sequence is not changed. Alternatively, the substitutions may be non-conservative, such that they are replaced by those having different properties which in turn affect the nature and properties of the sequence.

The skilled person will be aware of the fact that several different computer programs are available to determine the homology between two sequences. For instance, a comparison of sequences and determination of percent identity between two sequences may be accomplished using a mathematical algorithm. In a preferred embodiment, the percent identity between two amino acid sequences is determined using the Needleman and Wunsch (J. Mol. Biol. (48): 444-453 (1970)) algorithm which has been incorporated into the GAP program in the GCG software package (available on the internet at the accelrys website, more specifically at http://www.accelrys.com), using either a Blossom 62 matrix or a PAM250 matrix, and a gap weight of 16, 14, 12, 10, 8, 6 or 4 and a length weight of 1, 2, 3, 4, 5 or 6. The skilled person will appreciate that all these different parameters will yield slightly different results but that the overall percentage identity of two sequences is not significantly altered when using different algorithms.

In yet another embodiment, the percent identity between two nucleotide sequences is determined using the GAP program in the GCG software package (available on the internet at the accelrys website, more specifically at http://www.accelrys.com), using a NWSgapdna.CMP matrix and a gap weight of 40, 50, 60, 70 or 80 and a length weight of 1, 2, 3, 4, 5 or 6. In another embodiment, the percent identity between two amino acid or nucleotide sequences is determined using the algorithm of E. Meyers and W. Miller (CABIOS, 4: 11-17 (1989) which has been incorporated into the ALIGN program (version 2.0) (available on the internet at the vega website, more specifically ALIGN - IGH Montpellier, or more specifically at http://vega.igh.cnrs.fr/bin/align-guess.cgi) using a PAM120 weight residue table, a gap length penalty of 12 and a gap penalty of 4.

The nucleic acid and protein sequences of the present invention may further be used as a "query sequence" to perform a search against public databases to, for example, identify other family members or related sequences. Such searches may be performed using the BLASTN and BLASTX programs (version 2.0) of Altschul, et al., (1990) J. Mol. Biol. 215:403-10. BLAST nucleotide searches may be performed with the BLASTN program, score=100, word length=12 to obtain nucleotide sequences homologous to the nucleic acid molecules of the present invention. BLAST protein searches may be performed with the BLASTX program, score=50, word length=3 to obtain amino acid sequences homologous to the protein molecules of the present invention. To obtain gapped alignments for comparison purposes, Gapped BLAST may be utilized as described in Altschul et al., (1997) Nucleic Acids Res. 25 (17): 3389-3402. When utilizing BLAST and Gapped BLAST programs, the default parameters of the respective programs (e.g., BLASTX and BLASTN) may be used. (Available on the internet at the ncbi website, more specifically at www.ncbi.nlm.nih.gov).

An expression cassette for use in the present invention may comprise a polynucleotide sequence, preferably a gene encoding a spinosyn enhancing gene and/or a gene encoding a spinosyn biosynthetic enzyme. In an embodiment, the spinosyn enhancing gene and a gene encoding a spinosyn biosynthetic enzyme are as defined herein. An expression cassette may optionally comprise a selectable marker. An expression cassette may optionally comprise one or more control sequences, including an origin of replication. An expression cassette may optionally comprise one or more sequences to enable integration (for example by recombination) into a host cell genome. The term "expression cassette" may be used interchangeably with terms such as expression vector and genetic construct.

Suitable expression vectors for use in the present invention include prokaryotic and eukaryotic vectors (e.g., plasmid, phagemid, or bacteriophage), include mammalian vectors and plant vectors. Suitable prokaryotic vectors include plasmids such as, but not limited to, those commonly used for DNA manipulation in *Actinomyces,* (for example pSET152, pOJ260, pIJ101, pJV1, pSG5, pHJL302, pSAM2, pKC1250). Such plasmids are disclosed by Kieser et al., ("Practical Streptomyces Genetics", 2000). Other suitable vectors can include plasmids such as those capable of replication in *E. coli* (for example, pBR322, ColE1, pSC101, PACYC 184, itVX, pRSET, pBAD (Invitrogen, Carlsbad, Calif.) and the like). Such plasmids are disclosed by Sambrook (cf. "Molecular Cloning: A Laboratory Manual," second edition, edited by Sambrook, Fritsch, & Maniatis, Cold Spring Harbor Laboratory, (1989)) and many such vectors are commercially available. *Bacillus* plasmids include pC194, pC221, pT127, and the like, and are disclosed by Gryczan (In: The Molecular Biology of the Bacilli, Academic Press, NY (1982), pp. 307-329). Suitable Streptomyces plasmids include pli101 (Kendall et al., J. Bacteriol. 169:4177-4183, 1987), and *Streptomyces* bacteriophages include but not limited to such as ψC31 (Chater et al., In: Sixth International Symposium on Actinomycetales Biology, Akademiai Kaido, Budapest, Hungary (1986), pp. 45-54). Pseudomonas plasmids are reviewed by John et al., (Rev. Infect. Dis. 8:693-704, 1986), and Izaki (Jpn. J. Bacteriol. 33:729-742, 1978). In an embodiment, an expression cassette for use in the present invention may comprise a polynucleotide sequence encoding a spinosyn enhancing gene, as defined herein, a transcription terminator and a selectable marker. Preferably, the spinosyn enhancing gene is a polynucleotide sequence encoding VHb or a fragment, derivative or variant thereof. Independently, the transcription terminator may be the aph transcription terminator sequence. Independently, the selectable marker may be apramycin resistance gene. Preferably, the expression cassette is as shown in SEQ ID NO. 1.

Suitable expression vectors for use in the present invention include prokaryotic and eukaryotic vectors (e.g., plasmid, phagemid, or bacteriophage), include mammalian vectors and plant vectors. Suitable prokaryotic vectors include plasmids such as, but not limited to, those commonly used for DNA manipulation in *Actinomyces,* (for example pSET152, pOJ260, pIJ101, pJV1, pSG5, pHJL302, pSAM2, pKC1250. Such plasmids are disclosed by Kieser et al., ("Practical Streptomyces Genetics", 2000). Other suitable vectors can include plasmids such as those capable of replication in *E. coli* (for example, pBR322, ColE1, pSC101, PACYC 184, itVX, pRSET, pBAD (Invitrogen, Carlsbad, Calif.) and the like). Such plasmids are disclosed by Sambrook (cf. "Molecular Cloning: A Laboratory Manual," second edition, edited by Sambrook, Fritsch, & Maniatis, Cold Spring Harbor Laboratory, (1989)) and many such vectors are commercially available. *Bacillus* plasmids include pC194, pC221, pT127, and the like, and are disclosed by Gryczan (In: The Molecular Biology of the Bacilli, Academic Press, NY (1982), pp. 307-329). Suitable Streptomyces plasmids include pli101 (Kendall et al., J. Bacteriol. 169:4177-4183, 1987), and *Streptomyces* bacteriophages include but not limited to such as ψC31 (Chater et al., In: Sixth International Symposium on Actinomycetales Biology, Akademiai Kaido, Budapest, Hungary (1986), pp. 45-54). Pseudomonas plasmids are reviewed by John et al., (Rev. Infect. Dis. 8:693-704, 1986), and Izaki (Jpn. J. Bacteriol. 33:729-742, 1978).

Suppression of the expression of particular genes is an important tool both for research and for the development of genetically engineered organisms more fitted for a particular purpose. Gene silencing can be accomplished by the introduction of a transgene corresponding to the gene of interest in the antisense orientation relative to its promoter (see, e.g., Sheehy et al., Proc. Nat'l Acad. Sci. USA 85:8805 8808 (1988); Smith et al., Nature 334:724 726 (1988)), or in the sense orientation relative to its promoter (Napoli et al., Plant Cell 2:279 289 (1990); van der Krol et al., Plant Cell 2:291 299 (1990); U.S. Pat. No. 5,034,323; U.S. Pat. No. 5,231,020; and U.S. Pat. No. 5,283,184), both of which lead to reduced expression of the transgene as well as the endogenous gene.

Posttranscriptional gene silencing has been reported to be accompanied by the accumulation of small (20 to 25 nucleotide) fragments of antisense RNA, which can be synthesized from an RNA template and represent the specificity and mobility determinants of the process (Hamilton & Baulcombe, Science 286:950 952 (1999)). It has become clear that in a range of organisms the introduction of dsRNA (double-stranded RNA) is an important component leading to gene silencing (Fire et al., Nature 391:806 811 (1998); Timmons & Fire, Nature 395:854 (1998); WO99/32619; Kennerdell & Carthew, Cell 95:1017 1026 (1998); Ngo et al., Proc. Nat'l Acad. Sci. USA 95:14687 14692 (1998); Waterhouse et al., Proc. Nat'l Acad. Sci. USA 95:13959 13964 (1998); WO99/53050; Cogoni & Macino, Nature 399:166 169 (1999); Lohmann et al., Dev. Biol. 214:211 214 (1999); Sanchez-Alvarado & Newmark, Proc. Nat'l Acad. Sci. USA 96:5049 5054 (1999)). In bacteria the suppressed gene does not need to be an endogenous bacterial gene, since both reporter transgenes and virus genes are subject to posttranscriptional gene silencing by introduced transgenes (English et al., Plant Cell 8:179 188 (1996); Waterhouse et al., supra). However, in all of the above cases, some sequence similarity may be preferred between the introduced transgene and the gene that is suppressed.
The terms "host cell" and "recombinant host cell" are used interchangeably herein. Such terms refer not only to the particular subject cell, but also to the progeny or potential progeny of such a cell. Because certain modifications may occur in succeeding generations due to either mutation or environmental influences, such progeny may not, in fact, be identical to the parent cell, but are still included within the scope of the term as used herein.

The methods of the invention may include the additional steps of
a) culturing a genetically modified cell of the invention, under conditions appropriate for production of spinosyn; and
b) collecting spinosyn from a culture of the cell.

The examples which follow are set forth to illustrate the present invention, and are not to be construed as limiting thereof.

The present invention is explained in greater detail in the following non-limiting examples.

### EXAMPLES

### Identification of cosmid clones carrying the obscurin PKS gene obsA

Screening of a cosmid library was completed using an 806-bp probe from the *obsA* genomic locus of *S. spinosa* (SEQ ID NO: 8). A cosmid library was constructed from a *S*. *spinosa* strain by BioS&T (Montreal, Canada) using Supercos I cosmid vector (Stratagene, La Jolla, CA) and the genomic DNA from the *S. spinosa* strain which was prepared according to the genomic DNA isolation protocol described by Kieser et al., (2000).

The screening of the library with the *obsA* probe allowed for the identification of cosmid clones carrying the 5' end of *obsA* under stringent conditions. Probe labeling, hybridization and detection were carried out using DIG DNA Labeling Kit and the DIG Nucleic Acid Detection Kit (Roche, Basel, Switzerland) according to manufacturer's instructions. Specifically, the 806-bp PCR fragment derived from the 5' end of *obsA* was labeled using the Random Prime Labeling Kit (Roche). Hybridization was carried out overnight at 58°C in the hybridization oven followed by washing both filters twice at 65°C for a total of 30 minutes using the wash buffer containing 0.1X SSC and 0.1% SDS. The hybridized probe was detected using Roche's DIG Nucleic Acid Detection kit based on the enzyme-linked immunoassay with a highly specific anti-DIG-AP antibody conjugate and the color substrates NBT (nitro blue tetrazolium) and BCIP (5-Bromo-4-chloro-3-indolyl phosphate). Several sets of cosmids with strong hybridization signals were identified. Four of the clones, 1E3, 2N14, 3M23 and 4E16, were selected for cosmid DNA isolation and sequencing confirmation.

PCR Amplification of the cosmid clones using *obsA* Forward Primer (SEQ ID NO:9 5'-CAAGATCGTTGGGACCTGGCC-3') and *obsA* Reverse Primer (SEQ ID NO:10 5'-TCGACGTACTGGACCTCGGC-3') resulted in a single PCR fragment equivalent to the 806-bp probe in size. PCR reactions were completed according to manufacturer's instruction using the FAILSAFE™ PCR System (Epicentre Biotechnologies, Madison, WI).

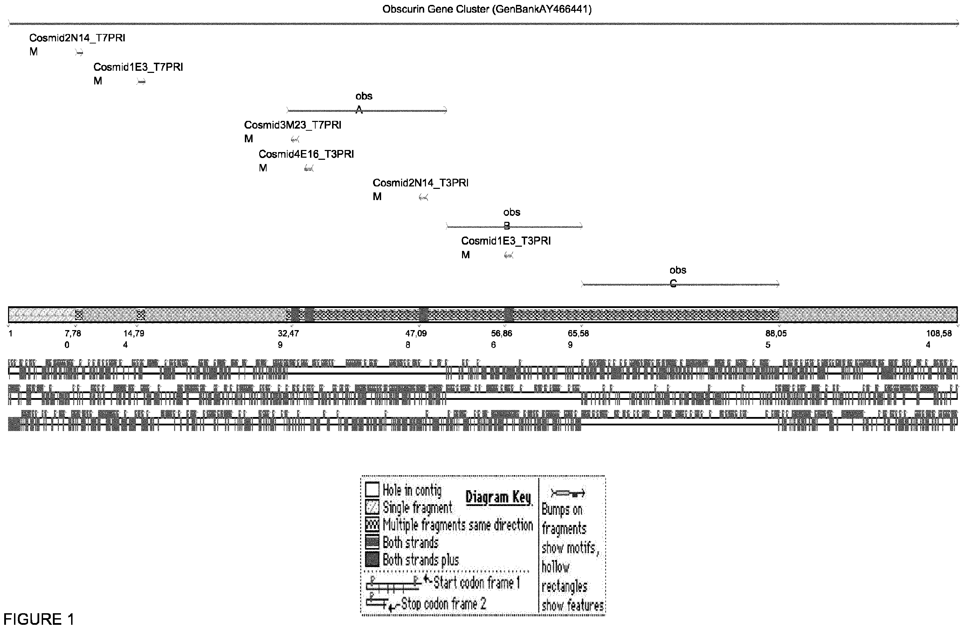

The DNA inserts carried by the four cosmid clones were also sequenced at both ends in order to map the cosmid clones onto the obscurin gene cluster and to estimate the insert size of the cosmid clones (Figure 1). Cosmid clone 1E3 was determined to be 42,900 bp in size and contains the 5' end of *obsA* in the middle of the DNA insert. Cosmid clone 2N14 was determined to be 40,153 bp in size and does not carry the entire *obsA* coding region. The inserts in cosmid clones 3M23 and 4E16 contain the 5' end of *obsA* at one end and the DNA sequences beyond the obscurin gene cluster at the other end. The actual sizes of cosmid clones 3M23 and 4E16 were not estimated. Cosmid clone 1E3 was chosen for completing *obsA* disruption to determine the impact of polyketide synthase gene function abolishment on *S. spinosa* characteristics and spinosyn production.

### Engineering the obsA disruption cosmid clone via PCR targeting

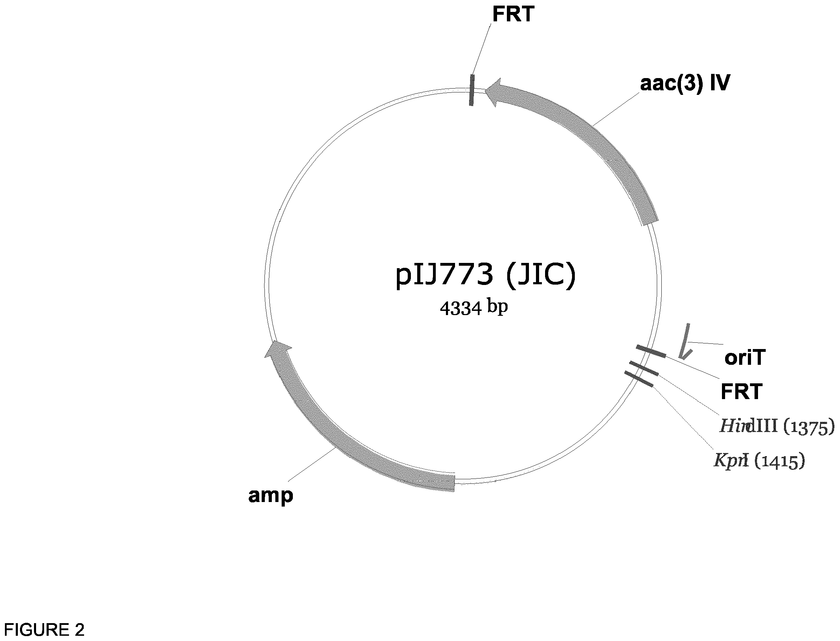

To disrupt *obsA* within the obscurin gene cluster, the apramycin resistance disruption cassette (FRT*-aac3(IV)-oriT*-FRT) of plasmid pIJ773 (provided by the John Innes Center Plant Biosciences Limited, Norwich, England; Figure 2) was integrated into cosmid clone 1E3 via PCR targeting.

Integration of the *obsA* disruption cassette (FRT*-aac3(IV)-oriT*-FRT) into cosmid clone 1E3 was carried out according to Gust et al., (2002) with the following modifications. The *E. coli* BW25141/pKD78 acquired from The Coli Genetic Stock Center (CGSC) at Yale University which contains the lambda red recombinase expression plasmid pKD78, derived from pKD46 (Datsenko and Wanner, 2000) was used. The following long PCR primers, ObsA 5' FRT aac3 (SEQ ID NO: 11 5' - GGCAATGCGCAGAGTTCGTAGTGCGGGAGCCATTTGATGTGTAGGCTGG AGCTGCTTC - 3') and ObsA internal oriT FRT (SEQ ID NO: 12 5' - GAAGAAGGCGGCGTCGAACTGGTCGACCTCGGTGAGGAAATTCCGGGG ATCCGTCGACC - 3'), were used for amplification of the 1322-bp fragment carrying FRT-*aac3(IV)-oriT*-FRT using pIJ773 (Figure 2) as template. The amplified fragment of 1,322 bp was purified and integrated into cosmid 1E3 according to Gust et al., (2002).

All ten of the recombinant *E. coli* clones which were produced from the PCR targeting were resistant to apramycin and had the same *BamHI* restriction enzyme digestion pattern (this digestion pattern is different from that of cosmid clone 1E3 not containing the disruption cassette). Further confirmation of the recombinant clones was carried out via amplification using a pair of primers annealing to the DNA regions 95 bp upsteam of the start codon of *obsA* and 334 bp downstream of the *obsA* start codon, respectively. The primers are obsA 5' upstream forward (SEQ ID NO:12 5'-CGACCGGTGTGTCGATGTTAGGGT-3') and obsA internal reverse (SEQ ID NO:14 5'-CTTCCAACGCTTCCCAGCCC-3'). PCR reactions were completed according to manufacturer's instruction using the FAILSAFE™ PCR System (Epicentre Biotechnologies, Madison, WI).

Amplification using either the genomic DNA of the spinosad strain used to created the cosmids or the DNA from cosmid clone 1E3 yielded the expected fragment of 429 bp. Amplification using the cosmid DNA isolated from nine (clone 10 was not pursued due to redundancy) of the ten recombinant cosmid clones yielded a single fragment corresponding to the expected size of 1,538 bp. The expected PCR fragment of 1,538 bp is due to the insertion of 1,322 bp of the disruption cassette (FRT-*aac3(IV)-oriT*-FRT) at the 5' end of *obsA* with simultaneous deletion of 213 bp from the *obsA* gene. All of the recombinant cosmid clones produced a single PCR fragment of 1,538 bp and did not produce the 429-bp fragment seen in the control cosmid clone 1E3 indicating that each of the recombinant cosmid clones contained the disruption cassette (FRT-*aac3(IV)-oriT*-FRT) at the 5' end of *obsA.*

### Disruption of obsA within the obscurin gene cluster via integration of the disruption cassette (FRT-aac3(IV)-oriT-FRT) in S. spinosa

Conjugation methods were used to introduce the recombinant cosmid clone carrying the *obsA* disruption cassette (FRT-*aac3(IV)-oriT*-FRT) into *S. spinosa* strains in order to achieve transconjugants from all of the target strains for conclusive analysis of the impact of *obsA* disruption on growth and spinosyn production.

Mycelial conjugation between the donor strain carrying the recombinant cosmid 1E3 and a recipient *S. spinosa* strain, NRRL 18538, was carried out according to the method described by Matsushima et al., (1994).

Transconjugants were produced. Nearly all of the primary transconjugants confirmed the desired apramycin-resistant phenotype indicating that the transconjugants carried the apramycin resistance gene, *aac3(IV),* integrated into the obscurin polyketide synthase gene cluster via homologous recombination. Several select transconjugants were tested for the presence of the DNA fragment corresponding to the size of a 785 bp *aac3(IV)* fragment upon PCR amplification. The negative controls which did not contain the disruption cassette (FRT-*aac3(IV)-oriT*-FRT) did not produce a PCR amplicon.

### The impact of obsA disruption on S. spinosa growth and spinosyn production

The impact of *obsA* disruption on spinosyn production was evaluated using a spinosyn shake flask protocol. Fermentation of the transconjugants was performed under conditions described by Burns et al., (WO 2003070908). Analysis of the fermentation broth for the presence of spinosyn factors was carried out under conditions described by Baltz et al., (US Patent No. 6,143,526).

Direct comparison of the performance of the transconjugants relative to their respective parent strains was achieved for the *S. spinosa* isolates. Four of the *obsA* knockout mutants derived from strain NRRL 18538 were evaluated in shake flasks. The average titer of each knockout mutant was higher than the parent strain. However, the differences did not appear to be significant based on statistical analysis (Table 1).

**Table 1. Spinosyn production at day 10 by the obsA knockout mutants derived from NRRL 18538.**

| Strain | Major Spinosyn Factors | Spinosyn Titers Relative to Control in Percentage at Day 10 of Fermentation |
|---|---|---|
| NRRL 18538 Parent | A/D | 100 |
| NRRL 18538 Δ*obsA*-1 | A/D | 108 |
| NRRL 18538 Δ*obsA*-2 | A/D | 114 |
| NRRL 18538 Δ*obsA*-3 | A/D | 108 |
| NRRL 18538 Δ*obsA*-6 | A/D | 107 |

The disruption of the obscurin PKS gene *obsA* in the A/D strain, NRRL 18538, had no negative impact on spinosyn production as compared to the respective parent control strains under current shake flask fermentation conditions. Lack of negative impact on spinosyn production upon *obsA* disruption qualifies the obscurin polyketide synthase gene cluster as a neutral site for integration and expression of target genes of interest for improved spinosyn production and fermentation processes. This genomic locus serves as an example of an integration site for the integration of genes within the genome of *S. spinosa.*

### Construction of a Hemoglobin Gene Expression Cassette

A hemoglobin gene expression cassette was designed to contain the following key features: i) the apramycin resistance gene (originally from *Klebsiella pneumoniae* under GenBank Accession Number X99313; Kieser et al., 2001) promoter (*aac3(IV) promoter)* which is functional in *S. spinosa;* ii) codon optimized *Vitreoscilla* hemoglobin gene *VHb* (Table 1) designed using a *S*. *spinosa* codon preference table and GeneDesigner software (DNA 2.0, Menlo Park, California); and iii) the *aph* transcription terminator sequence (Pulido and Jiménez, 1987). The selection and arrangement of these gene elements ensure stable *VHb* transcription and optimum protein expression, thereby providing robust expression of the hemoglobin protein. The hemoglobin gene expression cassette (SEQ ID NO:1) was synthesized by DNA 2.0 (Menlo Park, CA). The resulting 977 bp synthetic *HinDIII* flanked fragment was cloned into the pJ201 vector (Figure 1) and was sequenced by DNA2.0 to confirm sequence accuracy prior to shipment. The resulting plasmid was designated as pDAB109000.

**Table 2. Comparison of Vitreoscilla hemoglobin gene (VHb) codons and the synthetic hemoglobin gene preferred codons for optimal expression in S. spinosa. The synthetic VHb codons are preferred in S. spinosa and were selected to replace the preferred codons from the Vitreoscilla hemoglobin gene using the GeneDesigner software.**

| Amino Acid | **Leu** | **Arg** | **Thr** | **Ile** | **Lys** | **Ala** | **Val** | **Pro** | **His** |
|---|---|---|---|---|---|---|---|---|---|
| *Vitreoscilla VHb* codon | TTA | CGT | ACC | ATT | AAA | GCC | GTT | CCT | CAT |
| | TTG | CGC | ACT | | | GCT | GTA | CCA | |
| Amino Acid | **Leu** | **Arg** | **Thr** | **Ile** | **Lys** | **Ala** | **Val** | **Pro** | **His** |
| | | | | | | GCA | GTC | | |
| Synthetic *VHb* codon | CTG | CGG | ACG | ATC | AAG | GCG | GTG | CCG | CAC |
| Amino Acid | **Asp** | **Gly** | **Ser** | **Asn** | **Cys** | **Phe** | **Gln** | **Glu** | **Tyr** |
| *Vitreoscilla VHb* codon | GAT | GGT | TCT | AAT | TGT | TTT | CAA | GAA | TAT |
| Synthetic *VHb* codon | GAC | GGC | TCG | AAC | TGC | TTC | CAG | GAG | TAC |

### Introduction of the Hemoglobin Gene Expression Cassette into Cosmid Clone 1E3

The *obscurin* gene cluster was chosen as the site for integration of the hemoglobin gene expression cassette because disruption of the *obscurin* gene cluster does not reduce spinosyn production. The hemoglobin gene expression cassette was cloned into cosmid 1E3 (which contains a genomic DNA partial *obscurin* gene cluster). This cosmid was engineered to facilitate stable integration of the hemoglobin gene expression cassette within the *obscurin* gene cluster of the genome *of S. spinosa.*

The hemoglobin gene expression cassette was first cloned into vector pIJ773 (John Innes Centre, Norwich, UK) via a conventional cloning method. This cassette, in addition to the apramycin resistance gene and *oriT* flanked by FRT recombination sequences was then cloned into cosmid 1E3 via PCR targeting (Gust et al., 2002). Briefly, the hemoglobin gene expression cassette was first amplified using pDAB109000 as a template and the following primers, aac3-VHb Forward (SEQ ID NO:2 5'-CGCTGAAAAGCTTCTGACGCCG-3') and aac3-VHb Reverse (SEQ ID NO:3 5'-CAACCCGTACCACGGCCTCT-3'). The amplified PCR fragment was digested with *HindIII*/*KpnI* to generate two fragments; a 864 bp *HindIII*/*KpnI* fragment and a 83 bp *HindIII*/*KpnI* fragment. The 864 bp *HindIII*/*KpnI* fragment, carrying the hemoglobin gene expression cassette including the transcription terminator, was gel purified and cloned into vector pIJ773 which had been digested with *HindIII*/*KpnI.* A total of 38 transformants grown on Luria-Bertani media (LB) containing 50 µg/mL of apramycin were selected for further analysis and confirmation. After overnight growth in liquid LB containing apramycin at 50 µg/mL, cells were harvested by centrifugation at 13,000 RPM in a microcentrifuge for 2 minutes.

Cell pellets carrying the control plasmid pDAB109000 produced a reddish color indicating the presence of the hemoglobin protein. However, only seven of the recombinant clones produced visible reddish cell pellets ranging from light red to a more intense red color. These seven clones were selected and advanced to the next step which included plasmid DNA isolation and restriction enzyme digestions. Two of the seven clones, clone 22 and clone 30, produced the desired restriction patterns based on restriction analysis using the following enzymes *NdeI, HindIII*/*KpnI* and *NdeI*/*KpnI.* The hemoglobin gene region of both clones and the PCR fragment carrying the hemoglobin gene expression cassette were sequenced. The hemoglobin gene region of clone 22 and the PCR fragment was completely sequenced and the sequence data confirmed that the desired synthetic hemoglobin gene was amplified without any errors and was cloned into pIJ773 (Figure 2). The resulting plasmid was labeled as pDAB109001.

Integration of the FRT :: *aac3(IV)* gene expression cassette :: *oriT*:: FRT :: synthetic hemoglobin gene expression cassette into cosmid clone 1E3 was carried out as described via a modified PCR Targeting protocol (Gust et al., 2002). The following modifications were incorporated into the protocol: i) the host *E. coli* for introducing the recombinant cosmid clone carrying the hemoglobin gene expression cassette was *E. coli* BW25141/pKD78 acquired from The Coli Genetic Stock Center (CGSC) at Yale University; ii) lambda red recombinase expression plasmid pKD78, derived from pKD46 (Datsenko and Wanner, 2000); and iii) the following PCR primers, obsA Internal Strep stem loop (SEQ ID NO:4 5'-GAAGAAGGCGGCGTCGAACTGGTCGACCTCGGTGAGGAAGGTACCTC CGACCGCACGGC-3') and obsA 5' FRT aac3 (SEQ ID NO:5 5'-GGCAATGCGCAGAGTTCGTAGTGCGGGAGCCATTTGATGTGTAGGCT GGAGCTGCTTC-3') were designed and used. The resulting amplification produced a 2,216 bp fragment consisting of (FRT :: *aac3(IV) :: oriT::* FRT :: *VHb*) within cosmid clone 1E3.

Integration within cosmid clone 1E3 was designed to occur at the 5' end of *obsA,* resulting in the removal of 600 bp of the *obsA* coding sequence. This ensures complete disruption of the first polyketide synthase enzyme within the *obscurin* gene cluster. Once integrated, the hemoglobin gene expression cassette was orientated in such a way that its coding sequence was in the opposite orientation of the *obsA* coding sequence. This directionality prevents transcription readthrough from the putative *obsA* promoter which could potentially result in undesired transcription. Putative recombinant clones carrying the (FRT :: *aac3(IV) :: oriT ::* FRT :: *VHb*) fragment were isolated and confirmed by restriction analysis. Restriction digestion using *BamHI* indicated that the recombinant cosmid clones carrying the hemoglobin gene cassette had different restriction patterns as compared to the parent cosmid clone, 1E3. Moreover restriction digestion using *NdeI* and *ClaI,* intended to re-generate the synthetic hemoglobin gene coding sequence, revealed that only the three recombinant clones produced a the 444 bp fragment carrying the entire coding region of the synthetic hemoglobin gene.

### Conjugation of the (FRT :: aac3(IV) :: oriT :: FRT :: VHb) Gene Expression Cassette into S. spinosa strains

Mycelial conjugation between the donor strain, *Escherichia coli* S17, containing the FRT :: *aac3(IV)* gene expression cassette :: *oriT* :: FRT :: synthetic hemoglobin gene expression cassette within cosmid 1E3 and the spinosyn producing strain, DAS-2, was completed. Mycelial conjugation between the donor strain and the recipient *S. spinosa* strain was carried out according to the method described by Matsushima et al., (1994). Colonies initially identified as resistant to apramycin and nalidixic acid on the conjugation media were patched onto Brain Heart Infusion agar media (BHI) containing 50 µg/mL of apramycin and 25 µg/mL of nalidixic acid to confirm the antibiotic resistance phenotype of the colonies. Colonies having the desired antibiotic resistance phenotype were presumed to contain the FRT :: *aac3(IV):: oriT* :: FRT :: *VHb* cassette integrated within the *obsA* locus of the *obscurin* gene cluster. To confirm the presence of the apramycin resistance gene, *aac3(IV),* genomic DNA of the putative transconjugants was isolated using the PURELUTE™ Bacterial Genomic Kit (Edge BioSystems, Gaithersburg, MD) according to manufacturer's instructions. This genomic DNA (gDNA) was used as template for PCR amplification size confirmation of a 785 bp *aac3(IV)* DNA fragment using the FAILSAFE™ PCR System (Epicentre Biotechnologies, Madison, WI).

### Confirmation of the Presence of a Hemoglobin Gene in Transconjugants

To confirm that the synthetic hemoglobin gene was present in the transconjugants, the genomic DNA of a select number of transconjugants was isolated (via the PURELUTE™ Bacterial Genomic Kit) and used as template for PCR amplification of the hemoglobin gene (via the FAILSAFE™ PCR System). Recombinant strains produced a PCR product of 395 bp, corresponding to the size of the hemoglobin gene coding region intended for amplification. The PCR fragments were purified and DNA sequencing of both strands was completed to further confirm the presence of the *VHb* gene. Alignment of the sequence derived from the PCR fragments with the synthetic hemoglobin gene sequence synthesized by DNA2.0 indicated that these recombinant strains carried the hemoglobin gene with sequence identical to the designed DNA sequence.

### Hemoglobin Gene Expression Under Fermentation Conditions

Expression of the synthetic hemoglobin gene in the recombinant strain carrying the hemoglobin gene expression cassette under fermentation conditions was determined. Recombinant strains containing the *VHb* expression cassette were grown under shake flask fermentation conditions, and the corresponding total RNA samples were isolated via the RIBOPURE™-Bacteria kit (Ambion, Austin, TX) and prepared for use as template in the RT-PCR assay. Fermentation of the double crossover mutant was performed under conditions described by Burns et al., (WO 2003070908). Analysis of the fermentation broth for the presence of spinosyn factors can be carried out under conditions described by Baltz et al., (US Patent No. 6,143,526). To confirm the presence of the spinosyn factors in the supernatant, extracts of the fermentation broth were dried down in a SpeedVac overnight followed by partition of the residue between water and ether. The ether layer was dried by evaporation under N₂ stream. The sample was then dissolved in acetone-d₆ and transferred to an NMR tube for 1D proton NMR acquisition. The NMR profiles were compared to those of spinosyn standards.

Total RNA preparation was treated with DNaseI (Ambion, Austin, TX) immediately following RNA purification according to manufacturer's instructions. RT-PCR of the synthetic hemoglobin gene using the isolated total RNA was carried out using OneStep RT-PCR kit (Qiagen, Valencia, CA) according to manufacturer's directions. Primers for amplification of the coding region of the synthetic hemoglobin gene were SynHemo Forward (SEQ ID NO:6 5'-CAGACGATCAACATCATCAAGGCG-3') and SynHemo Reverse (SEQ ID NO:7 5'-ACCTGGATGAACACGTCCGC-3'). The recombinant strains containing the *VHb* expression cassette produced a specific fragment of 395 bp corresponding to the hemoglobin gene coding region intended for amplification. In the same assay, total RNA isolated from the non-recombinant control strains did not produce a PCR fragment corresponding in size to the synthetic hemoglobin gene. These results confirmed that a synthetic hemoglobin gene, driven by the apramycin resistance gene promoter, was transcribed and expressed in *S. spinosa* under fermentation conditions.

### Effect of Hemoglobin Gene Expression on Spinosad Production

The impact of the hemoglobin gene expression on spinosad (A/D) production was evaluated by growing the recombinant strains under shake flask fermentation conditions as described above. The fermentation results were compared to spinosyn A/D levels produced in the respective parent strain, which does not carry the hemoglobin gene. The number of recombinant strains selected for evaluation in shake flasks was based on the number of transconjugants available for testing and the desire to obtain data which would determine whether the expression of the synthetic hemoglobin gene produces increased levels of spinosad A/D.

Seven recombinant strains derived from DAS-2 were tested. All of which outperformed the parent strain, DAS-2, in spinosad production. The parent strain accumulated spinosad within the expected range. The statistically significant increase in spinosad A/D production ranged from 4.6% to 12.9% for the strains which contained the *VHb* gene (Table 2). The consistency among the recombinant strains in outperforming the control strain in spinosad production under shake flask fermentation conditions resulted in a significant percent increase of spinosad A/D titers. This increase in spinosad titers indicated that the recombinant strains as a whole have acquired enhanced spinosad production capability due to the presence of the synthetic hemoglobin gene in the genome. Expression of the synthetic hemoglobin gene under shake flask fermentation conditions indicates that the expression of the hemoglobin gene increases spinosad production.

**Table 3: Comparison of DAS-2 and DAS-2 VHb recombinant strain containing a chromosomally integrated VHb gene in spinosad production.**

| Strain ID | Major Spinosyn Factors | Spinosyn Titers Relative to Control in Percentage at Day 10 of Fermentation |
|---|---|---|
| DAS-2 | A/D | 100.0 |
| DAS-2 VHb1 | A/D | 107.4 |
| DAS-2 VHb4 | A/D | 104.6 |
| DAS-2 VHb5 | A/D | 108.0 |
| DAS-2 VHb6 | A/D | 110.5 |
| DAS-2 VHb7 | A/D | 112.9 |
| DAS-2 VHb8 | A/D | 110.4 |
| DAS-2 VHb9 | A/D | 105.6 |

Based on the fermentation results reported above, the following recombinant strains, DAS-2 VHb7 and DAS-2 VHb8, were cryogenically preserved in 20% glycerol using actively growing vegetative cultures. A dedicated shake flask fermentation study was carried out to: i) evaluate the cryo preserved strains; ii) ensure reproducibility of the fermentation results; and iii) provide evidence for advancement of select strains for future work. In this study the control strains produced spinosad at the expected levels, and the entire study was conducted without any unexpected deviations from the operating protocol. Each recombinant strain outperformed both control strains during and at the end of the fermentation cycle confirming the initial observations from the shake flask fermentation studies. The increase in spinosad levels relative to the control strains from the recombinant strains was statistically significant.

The obscurin polyketide synthase gene cluster was used for integration and expression of a codon optimized hemoglobin gene in *S. spinosa* strain, DAS-2. Integration and expression of the hemoglobin gene resulted in improved A/D production in the shake flask fermentation of *S. spinosa,* DAS-2.

The foregoing is illustrative of the present invention, and is not to be construed as limiting thereof. The invention is defined by the following claims, with equivalents of the claims to be included therein.
## Claims

1. A method for integrating a gene by homologous recombination into chromosomal DNA of a *S*. *spinosa* host cell, the method comprising the steps of:
a) cloning two genomic DNA fragments from the *obsA* gene of the obscurin polyketide synthase gene locus to generate an integration plasmid, wherein one genomic fragment is cloned upstream of a polynucleotide to be integrated, and the second genomic fragment is cloned downstream of a polynucleotide to be integrated, and the resulting ligated DNA fragment becomes the integration plasmid wherein upon integration the *obsA* gene of the obscurin polyketide synthase gene locus is disrupted by the polynucleotide;
b) introducing the plasmid of step (a) into the host cell;
c) integrating the polynucleotide into the native *obsA* gene of the obscurin polyketide synthase gene locus in the genome of the host cell; and,
d) screening the host cell for presence of said polynucleotide.

2. A method according to claim 1 wherein integration of the polynucleotide does not negatively impact *S. spinosa* growth or a desired metabolic characteristic.

3. A method for stably expressing a polynucleotide in an *S.spinosa* host cell comprising a method according to claim 1 or 2.

4. The method of any one of claims 1 to 3, wherein the polynucleotide comprises a gene expression cassette, wherein said gene expression cassette comprises a gene encoding a spinosyn biosynthetic enzyme and/or a gene encoding an oxygen binding protein.

5. The method of any one of claims 1 to 4 wherein the polynucleotide comprises a selectable marker.

6. A genetically modified *S. spinosa* host cell comprising an added gene in the chromosomal DNA at the PKS gene *obsA* of the obscurin polyketide synthase gene cluster or a substituted gene which is not identical to a native *S. spinosa* gene.

7. A genetically modified *S. spinosa* host cell according to claim 6, wherein the gene which has been added is identical to a native *S. spinosa* gene.

8. A method for production of a protein product of a target gene, the method comprising preparing a *S*. *spinosa* host cell in which a specific number of copies of said target gene are integrated into the genome of *S. spinosa* according to steps a) to d) of claim 1, and growing said cell in a suitable culture medium for sufficient time for said target gene to be expressed and said protein to be produced.

9. A method for stable expression of a target gene, the method comprising:
integrating into an *S. spinosa* genome, an obscurin polyketide synthase gene cluster as a neutral site for integration and expression of a target gene of interest;
integrating the target gene of interest into the neutral site whereby the obscurin polyketide synthase gene cluster is disrupted;
wherein the target gene of interest encodes an oxygen binding protein.

10. The method of claim 9, further wherein the obscurin PKS gene *obsA* of the integrated cluster is the site of integration.

11. A method according to claim 9 or 10 wherein the target gene comprises a coding sequence functionally linked to a heterologous promoter heterologous to the strain of *S. spinosa.*

12. A method according to any one of claims 9 to 11 wherein the target gene comprises a selectable marker.

13. A method according to any one of claims 9 to 12 wherein integration comprises homologous recombination.

14. A method according to any one of claims 9 to 13 further comprising:
culturing a host cell under conditions appropriate for production of spinosyn;
collecting spinosyn from a culture of the host cell.

15. An expression construct comprising the obscurin polyketide synthase gene *obsA,* wherein the obsA gene comprises a gene encoding an oxygen binding protein integrated therein.

16. An expression construct according to claim 15 wherein the expression construct is suitable for integration into a host cell genome.

17. Use of an expression construct according to claims 15 or 16 in a method for integrating a gene by homologous recombination into chromosomal DNA of a *S*. *spinosa* host cell, for stable gene expression.

18. A method for preparing a *S*. *spinosa* strain in which a specific number of copies of a chosen gene or of polynucleotide are integrated into a *S*. *spinosa* genome, wherein said method comprises:
a) cloning an obscurin polyketide synthase gene locus into a mobile element, wherein there is at least one marker gene located within the mobile element;
b) integrating said mobile element into an obscurin polyketide synthase gene locus in the chromosome or episome of said *S. spinosa* strain;
c) complementing said mobile element with a functional gene;
d) expressing said functional mobile element gene;
e) selecting said strain on a medium that is selectable for said marker gene in a manner such that a strain containing a specific number of copies of the obscurin polyketide synthase gene locus is obtained;
f) recovering the strain thereby selected; and
g) integrating a polynucleotide at the specific number of copies of the obscurin polyketide synthase gene locus at the obscurin PKS gene *obsA,* whereby the obscurin polyketide synthase gene locus is disrupted.

19. The method of claim 18, wherein the chromosome or episome is contained on a plasmid and is introduced by transformation.

20. The method according to claim 18 or 19 wherein the mobile element is a genetic construct.

21. The method, cell or construct of any one of the preceding claims, wherein integration of a polynucleotide does not negatively impact spinosyn production, growth, or other desired metabolic characteristics.

22. Use of a genetically modified *S. spinosa* host cell comprising an added, substituted or deleted gene in the chromosomal DNA at the obscurin polyketide synthase gene cluster in a method of stable gene expression.

23. Use according to claim 22 wherein the host cell is as defined in any one of claims 6 or 7.

24. Use according to claim 22 or 23, wherein the method comprises culturing the host cell under conditions appropriate for production of spinosyn; collecting spinosyn from a culture of the host cell.

## Patentansprüche

1. Verfahren zur Integration eines Gens in chromosomale DNA einer *S. spinosa-Wirtszelle* durch homologe Rekombination, wobei das Verfahren die folgenden Schritte umfasst:
a) Klonen zweier genomischer DNA-Fragmente vom *obsA*-Gen des Obscurin-Polyketidsynthase-Genlocus, um ein Integrationsplasmid zu erzeugen, wobei ein genomisches Fragment stromaufwärts von einem zu integrierenden Polynukleotid geklont wird, und das zweite genomische Fragment stromabwärts von einem zu integrierenden Polynukleotid geklont wird, und wobei das resultierende ligierte DNA-Fragment das Integrationsplasmid wird, wobei nach der Integration das *obsA*-Gen des Obscurin-Polyketidsynthase-Genlocus durch das Polynukleotid unterbrochen ist;
b) Einführen des Plasmids von Schritt (a) in die Wirtszelle;
c) Integrieren des Polynukleotids in das native *obsA*-Gen des Obscurin-Polyketidsynthase-Genlocus in das Genom der Wirtszelle; und,
d) Screenen der Wirtszelle auf Vorhandensein des Polynukleotids.

2. Verfahren nach Anspruch 1, wobei die Integration des Polynukleotids sich nicht negativ auf das *S*. *spinosa*-Wachstum oder eine gewünschte metabolische Eigenschaft auswirkt.

3. Verfahren zum stabilen Exprimieren eines Polynukleotids in einer S. *spinosa-*Wirtszelle*,* das ein Verfahren nach Anspruch 1 oder 2 umfasst.

4. Verfahren nach einem der Ansprüche 1 bis 3, wobei das Polynukleotid eine Genexpressionskassette umfasst, wobei die Genexpressionskassette ein Gen, das ein Spinosyn-Biosyntheseenzym kodiert und/oder ein Gen umfasst, das ein Sauerstoff bindendes Protein kodiert.

5. Verfahren nach einem der Ansprüche 1 bis 4, wobei das Polynukleotid einen auswählbaren Marker umfasst.

6. Genetisch veränderte *S*. *spinosa*-Wirtszelle, die ein hinzugefügtes Gen in der chromosomalen DNA beim PKS-Gen *obsA* des Obscurin-Polyketidsynthase-Genclusters oder ein substituiertes Gen umfasst, das nicht mit einem nativen *S*. *spinosa-*Gen identisch ist.

7. Genetisch veränderte *S*. *spinosa*-Wirtszelle nach Anspruch 6, wobei das Gen, das hinzugefügt wurde, mit einem nativen *S. spinosa*-Gen identisch ist.

8. Verfahren zur Herstellung eines Proteinprodukts eines Zielgens, wobei das Verfahren das Präparieren einer *S. spinosa*-Wirtszelle, in die eine bestimmte Anzahl von Kopien des Zielgens in das Genom der *S*. *spinosa* entsprechend den Schritten a) bis d) von Anspruch 1 integriert ist, und das Züchten der Zelle in einem geeigneten Kulturmedium für eine ausreichend lange Zeit, damit das Zielgen exprimiert und das Protein hergestellt werden kann, umfasst.

9. Verfahren zur stabilen Expression eines Zielgens, wobei das Verfahren Folgendes umfasst:
Integrieren eines Obscurin-Polyketidsynthase-Genclusters in ein *S*. *spinosa*-Genom als eine neutrale Stelle für die Integration und die Expression eines Zielgens von Interesse;
Integrieren des Zielgens von Interesse in die neutrale Stelle, wodurch der Obscurin-Polyketidsynthase-Gencluster unterbrochen wird;
wobei das Zielgen von Interesse ein Sauerstoff bindendes Protein kodiert.

10. Verfahren nach Anspruch 9, wobei ferner das Obscurin-PKS-Gen *obsA* des integrierten Clusters die Stelle der Integration ist.

11. Verfahren nach Anspruch 9 oder 10, wobei das Zielgen eine kodierende Sequenz umfasst, die funktional mit einem heterologen Promoter verbunden ist, der heterolog zum Stamm der *S*. *spinosa* ist.

12. Verfahren nach einem der Ansprüche 9 bis 11, wobei das Zielgen einen auswählbaren Marker umfasst.

13. Verfahren nach einem der Ansprüche 9 bis 12, wobei die Integration homologe Rekombination umfasst.

14. Verfahren nach einem der Ansprüche 9 bis 13, das ferner Folgendes umfasst:
Kultivieren einer Wirtszelle unter Bedingungen, die für die Herstellung von Spinosyn geeignet sind;
Sammeln von Spinosyn von einer Kultur der Wirtszelle.

15. Expressionskonstrukt, das das Obscurin-Polyketidsynthase-Gen *obsA* umfasst, wobei das obsA-Gen ein Gen umfasst, das ein darin integriertes, Sauerstoff bindendes Protein kodiert.

16. Expressionskonstrukt nach Anspruch 15, wobei das Expressionskonstrukt zur Integration in ein Wirtszellengenom geeignet ist.

17. Verwendung eines Expressionskonstrukts nach den Ansprüchen 15 oder 16 bei einem Verfahren zum Integrieren eines Gens in eine chromosomale DNA einer *S*. *spinosa*-Wirtszelle durch homologe Rekombination zur stabilen Genexpression.

18. Verfahren zum Präparieren eines *S*. *spinosa*-Stammes, in dem eine bestimmte Anzahl von Kopien eines ausgewählten Gens oder von Polynukleotid in ein *S*. *spinosa*-Genom integriert sind, wobei das Verfahren Folgendes umfasst:
a) Klonen eines Obscurin-Polyketidsynthase-Genlocus in ein mobiles Element, wobei mindestens ein Markergen vorhanden ist, das sich innerhalb des mobilen Elements befindet;
b) Integrieren des mobilen Elements in einen Obscurin-Polyketidsynthase-Genlocus im Chromosom oder im Episom des *S*. *spinosa*-Stammes;
c) Ergänzen des mobilen Elements mit einem funktionellen Gen;
d) Exprimieren des funktionalen mobilen Elementgens;
e) Auswählen des Stammes auf einem Medium, das für das Markergen in so einer Weise auswählbar ist, dass ein Stamm gewonnen wird, der eine bestimmte Anzahl von Kopien des Obscurin-Polyketidsynthase-Genlocus enthält;
f) Wiedergewinnen des dadurch ausgewählten Stammes; und
g) Integrieren eines Polynukleotids bei der bestimmten Anzahl von Kopien des Obscurin-Polyketidsynthase-Genlocus beim Obscurin-PKS-Gen *obsA,* wodurch der Obscurin-Polyketidsynthase-Genlocus unterbrochen wird.

19. Verfahren nach Anspruch 18, wobei das Chromosom oder das Episom auf einem Plasmid enthalten ist und durch Transformation eingeführt ist.

20. Verfahren nach Anspruch 18 oder 19, wobei das mobile Element ein Genkonstrukt ist.

21. Verfahren, Zelle oder Konstrukt nach einem der vorhergehenden Ansprüche, wobei die Integration eines Polynukleotids sich nicht negativ auf die Herstellung, das Wachstum oder andere gewünschte metabolische Eigenschaften des Spinosyns auswirkt.

22. Verwendung einer genetisch veränderten *S*. *spinosa*-Wirtszelle, die ein hinzugefügtes, ein substituiertes oder ein entferntes Gen in der chromosomalen DNA beim Obscurin-Polyketidsynthase-Gencluster in einem Verfahren zur stabilen Genexpression umfasst.

23. Verwendung nach Anspruch 22, wobei die Wirtszelle wie in einem der Ansprüche 6 oder 7 definiert ist.

24. Verwendung nach Anspruch 22 oder 23, wobei das Verfahren das Kultivieren der Wirtszelle unter für die Herstellung von Spinosyn geeigneten Bedingungen und das Sammeln von Spinosyn von einer Kultur der Wirtszelle umfasst.

## Revendications

1. Procédé d'intégration d'un gène par recombinaison homologue dans l'ADN chromosomique d'une cellule hôte *S. spinosa,* le procédé comprenant les étapes de :
a) clonage de deux fragments d'ADN génomique du gène *obsA* du locus génétique de la polycétide synthétase obscurine pour produire un plasmide d'intégration, un fragment génomique étant cloné en amont d'un polynucléotide à intégrer, et le second fragment génomique étant cloné en aval d'un polynucléotide à intégrer, et le fragment d'ADN ligaturé obtenu devient le plasmide d'intégration, lors de l'intégration le gène *obsA* du locus génétique de la polycétide synthétase obscurine étant interrompu par le polynucléotide ;
b) introduction du plasmide de l'étape (a) dans la cellule hôte ;
c) intégration du polynucléotide dans le gène *obsA* natif du locus génétique de la polycétide synthétase obscurine dans le génome de la cellule hôte ; et,
d) criblage de la cellule hôte pour la présence dudit polynucléotide.

2. Procédé selon la revendication 1 dans lequel l'intégration du polynucléotide n'a pas d'impact négatif sur la croissance de *S*. *spinosa* ou sur une caractéristique métabolique souhaitée.

3. Procédé d'expression stable d'un polynucléotide dans une cellule hôte *S*. *spinosa* comprenant un procédé selon la revendication 1 ou 2.

4. Procédé de l'une quelconque des revendications 1 à 3, dans lequel le polynucléotide comprend une cassette d'expression génétique, ladite cassette d'expression génétique comprenant un gène codant une enzyme de biosynthèse de la spinosyne et/ou un gène codant une protéine liant l'oxygène.

5. Procédé de l'une quelconque des revendications 1 à 4 dans lequel le polynucléotide comprend un marqueur sélectionnable.

6. Cellule hôte *S*. *spinosa* génétiquement modifiée comprenant un gène ajouté dans l'ADN chromosomique au gène PKS *obsA* du cluster de gènes de la polycétide synthétase obscurine ou un gène substitué qui n'est pas identique à un gène de *S*. *spinosa* natif.

7. Cellule hôte *S*. *spinosa* génétiquement modifiée selon la revendication 6, dans laquelle le gène qui a été ajouté est identique à un gène natif de *S*. *spinosa.*

8. Procédé de production d'un produit protéique d'un gène cible, le procédé comprenant la préparation d'une cellule hôte *S. spinosa* dans laquelle un nombre spécifique de copies dudit gène cible sont intégrées dans le génome de *S*. *spinosa* conformément aux étapes a) à d) de la revendication 1, et la culture de ladite cellule dans un milieu de culture approprié pendant un temps suffisant pour que ledit gène cible soit exprimé et que ladite protéine soit produite.

9. Procédé pour l'expression stable d'un gène cible, le procédé comprenant :
l'intégration dans un génome de *S. spinosa,* un cluster de gènes de la polycétide synthétase obscurine comme site neutre pour l'intégration et l'expression d'un gène cible d'intérêt ;
l'intégration du gène cible d'intérêt dans le site neutre par laquelle le cluster de gènes de la polycétide synthétase obscurine est interrompu ;
dans lequel le gène cible d'intérêt code une protéine liant l'oxygène.

10. Procédé de la revendication 9, dans lequel le gène de la PKS obscurine *obsA* du cluster intégré est en outre le site d'intégration.

11. Procédé selon la revendication 9 ou 10 dans lequel le gène cible comprend une séquence codante liée de manière fonctionnelle à un promoteur hétérologue, hétérologue vis à vis de la souche de *S. spinosa.*

12. Procédé selon l'une quelconque des revendications 9 à 11 dans lequel le gène cible comprend un marqueur sélectionnable.

13. Procédé selon l'une quelconque des revendications 9 à 12 dans lequel l'intégration comprend une recombinaison homologue.

14. Procédé selon l'une quelconque des revendications 9 à 13 comprenant en outre :
la culture d'une cellule hôte dans des conditions appropriées pour la production de spinosyne ;
la récupération de la spinosyne à partir d'une culture de la cellule hôte.

15. Construction d'expression comprenant le gène de la polycétide synthétase obscurine *obsA,* dans laquelle le gène obsA comprend un gène y étant intégré codant une protéine liant l'oxygène.

16. Construction d'expression selon la revendication 15, la construction d'expression étant appropriée pour l'intégration dans un génome de cellule hôte.

17. Utilisation d'une construction d'expression selon les revendications 15 ou 16 dans un procédé d'intégration d'un gène par recombinaison homologue dans l'ADN chromosomique d'une cellule hôte *S*. *spinosa,* pour une expression génétique stable.

18. Procédé de préparation d'une souche de *S*. *spinosa* dans laquelle un nombre spécifique de copies d'un gène choisi ou d'un polynucléotide sont intégrées dans un génome de *S. spinosa,* ledit procédé comprenant :
a) le clonage d'un locus génétique de la polycétine synthétase obscurine dans un élément mobile, au moins un gène marqueur étant situé dans l'élément mobile ;
b) l'intégration dudit élément mobile dans un locus génétique de polycétide synthétase obscurine dans le chromosome ou l'épisome de ladite souche de *S*. *spinosa ;*
c) la complémentation dudit élément mobile avec un gène fonctionnel ;
d) l'expression dudit gène d'élément mobile fonctionnel ;
e) la sélection de ladite souche sur un milieu qui est sélectionnable pour ledit gène marqueur d'une manière telle qu'une souche contenant un nombre spécifique de copies du locus génétique polycétide synthétase obscurine soit obtenue ;
f) la récupération de la souche ainsi sélectionnée ; et
g) l'intégration d'un polynucléotide au nombre spécifique de copies du locus génétique polycétide synthétase obscurine au gène de la PKS obscurine *obsA,* ce par quoi le locus génétique polycétide synthétase obscurine est interrompu.

19. Procédé de la revendication 18, dans lequel le chromosome ou l'épisome est contenu dans un plasmide et est introduit par transformation.

20. Procédé selon la revendication 18 ou 19 dans lequel l'élément mobile est une construction génétique.

21. Procédé, cellule ou construction de l'une quelconque des revendications précédentes, l'intégration d'un polynucléotide n'ayant pas d'impact défavorable sur la production de spinosyne, la croissance ou autres caractéristiques métaboliques souhaitées.

22. Utilisation d'une cellule hôte *S. spinosa* génétiquement modifiée comprenant un gène ajouté, substitué ou délété dans l'ADN chromosomique dans le cluster de gènes de la polycétide synthétase obscurine dans un procédé d'expression génétique stable.

23. Utilisation selon la revendication 22 dans laquelle la cellule hôte est telle que définie dans l'une quelconque des revendications 6 ou 7.

24. Utilisation selon la revendication 22 ou 23, dans laquelle le procédé comprend la culture de la cellule hôte dans des conditions appropriées pour la production de spinosyne ; la récupération de la spinosyne à partir d'une culture de la cellule hôte.


<110> Dow AgroSciences LLC
<120> INTEGRATION OF GENES INTO THE CHROMOSOME OF SACCHAROPOLYSPORA SPINOSA
<130> P202910EP
<150> US 13/100,220
   <151> 2011-05-03
<150> US 13/100,202
   <151> 2011-05-03
<160> 14
<170> PatentIn version 3.5
<210> 1
   <211> 977
   <212> DNA
   <213> Artificial Sequence
<220>
   <223> Hemoglobin Gene Expression Cassette
<400> 1
   aagcttgaat tcgatatcag cccggtacct cgccgccaac ccgtaccacg gcctctgacg 60
   gctccggcca agccgcggaa agcgggggcc gggcgccggt ctccaggcgg ccgggtacct 120
   ccgaccgcac ggccgcttcc ggagtggtcc ggaagcggcc tgcggagcgc cctgcggggc 180
   ggctatcgat tcactccacc gcctgcgcgt acaggtccgc ctccacctgg atgaacacgt 240
   ccgcgatcac gccgtacgcc ttgccccacg cgtccaggat gtcgtccgtc gccgcgtcgc 300
   ccagcacctc cttgatcgcg cccagcagct cctggcccac gatcgggtag tgcgccgccg 360
   ccacgcccgc ctggcagtgc ttcaccgcga tcttcttcac cgccggcagg atcgccggca 420
   ggttctcgat gttctgcgcc gccgccagca ccgtcatcgc cagcgccttc ggctgctcca 480
   gcgactcctg ccggcccatg tcgaacagcg gccgcacctc cgggtgcttc gcgaacaggt 540
   tcttgtagaa cgtggtcgtg atcgtcacgc cgtgctcctt cagcaccggc accgtcgcct 600
   tgatgatgtt gatcgtctgc tggtccagca tatgattgca ctccaccgct gatgacatca 660
   gtcgatcata gcacgatcaa cggcactgtt gcaaatagtc ggtggtgata aacttatcat 720
   ccccttttgc tgatggagct gcacatgaac ccattcaaag gccggcattt tcagcgtgac 780
   atcattctgt gggccgtacg ctggtactgc aaatacggca tcagttaccg tgagctgcat 840
   tttccgctgc ataaccctgc ttcggggtca ttatagcgat tttttcggta tatccatcct 900
   ttttcgcacg atatacagga ttttgccaaa gggttcgtgt agactttcct tggtgtatcc 960
   aacggcgtca gaagctt 977
<210> 2
   <211> 22
   <212> DNA
   <213> Artificial Sequence
<220>
   <223> aac3-vhb Forward Primer
<400> 2
   cgctgaaaag cttctgacgc cg 22
<210> 3
   <211> 20
   <212> DNA
   <213> Artificial Sequence
<220>
   <223> aac3-vhb Reverse Primer
<400> 3
   caacccgtac cacggcctct 20
<210> 4
   <211> 59
   <212> DNA
   <213> Artificial Sequence
<220>
   <223> obsA Internal Strep Stem Loop Primer
<400> 4
   gaagaaggcg gcgtcgaact ggtcgacctc ggtgaggaag gtacctccga ccgcacggc 59
<210> 5
   <211> 58
   <212> DNA
   <213> Artificial Sequence
<220>
   <223> obsA 5' FRT aac3 Primer
<400> 5
   ggcaatgcgc agagttcgta gtgcgggagc catttgatgt gtaggctgga gctgcttc 58
<210> 6
   <211> 24
   <212> DNA
   <213> Artificial Sequence
<220>
   <223> SynHemo Forward Primer
<400> 6
   cagacgatca acatcatcaa ggcg 24
<210> 7
   <211> 20
   <212> DNA
   <213> Artificial Sequence
<220>
   <223> SynHemo Reverse Primer
<400> 7
   acctggatga acacgtccgc 20
<210> 8
   <211> 806
   <212> DNA
   <213> Saccharopolyspora spinosa
<400> 8
   caagatcgtt gggacctggc cgcacgtttc gacgaggagc tttccgggcg ggggagcctg 60
   gggtcgggcc gtggtggttt cctcaccgag gtcgaccagt tcgacgccgc cttcttcggg 120
   atctcccctc gtgaagccgc cgaactcgac ccccggcagc ggctcacgct ggagctgggc 180
   tgggaagcgt tggaagacgc cgggatcgtg ccgggtgcgc tgcgcggtga acgggtcgcc 240
   gtgttcgtgg gcgcgatggg ggacgactac gccacgctga gcttcgccga cggcggctcc 300
   cagatcggtc actacacggc gaccggtgtg cagcggagca tgatcgccaa ccggttgtcg 360
   tacgtgctcg gtgtgcacgg cccgagcttc gtcgtcgact ccggtcagtc atcctccctg 420
   gttgcggttc acctcgccgt ggagagcctg cgccgcggcg agtcttcggc cgccctggtc 480
   ggcggggtca ccctgaacct ggctgcggag agcatggcgg ccacggccag gttcggcgcg 540
   ctttcgcccg acgggctctg cttcactttc gatgcacgcg cgaacggcta cgcacgcggt 600
   gaaggcggcg gtttcgccgt cctcaagccg ctgcacctcg cgctcggcga tggcgacgac 660
   atctactgcg tgatccgcgg gaccgccatg aacaacgacg gtggcggcga tgggctcacc 720
   gtcccgaacg agcgcgccca gcaggcggtg ctcgcggagg cgtaccggcg agcgggagtg 780
   gacccggccg aggtccagta cgtcga 806
<210> 9
   <211> 21
   <212> DNA
   <213> Artificial Sequence
<220>
   <223> obsA Forward Primer
<400> 9
   caagatcgtt gggacctggc c 21
<210> 10
   <211> 20
   <212> DNA
   <213> Artificial Sequence
<220>
   <223> obsA Reverse Primer
<400> 10
   tcgacgtact ggacctcggc 20
<210> 11
   <211> 58
   <212> DNA
   <213> Artificial Sequence
<220>
   <223> ObsA 5' FRT aac3 Primer
<400> 11
   ggcaatgcgc agagttcgta gtgcgggagc catttgatgt gtaggctgga gctgcttc 58
<210> 12
   <211> 59
   <212> DNA
   <213> Artificial Sequence
<220>
   <223> ObsA internal oriT FRT Primer
<400> 12
   gaagaaggcg gcgtcgaact ggtcgacctc ggtgaggaaa ttccggggat ccgtcgacc 59
<210> 13
   <211> 24
   <212> DNA
   <213> Artificial Sequence
<220>
   <223> obsA 5' upstream forward Primer
<400> 13
   cgaccggtgt gtcgatgtta gggt 24
<210> 14
   <211> 20
   <212> DNA
   <213> Artificial Sequence
<220>
   <223> obsA internal reverse Primer
<400> 14
   cttccaacgc ttcccagccc 20